# EUROPEAN PATENT APPLICATION

(11) **EP 2 869 069 A1**
(43) Date of publication of application: **06.05.2015**
(21) Application number: 14194788.7
(22) Date of filing: 12.11.2010
(51) Int. Cl.: G01N 33/50

(54) **Rational design of regenerative medicine products**

(30) Priority: 12.11.2009 US 260833 P
(62) Divisional of application: 10795492.7
(71) Applicant: Tengion, Inc., Winston-Salem, NC 27103 (US)
(72) Inventor: Presnell, Sharon C., San Diego, CA 92128-4330 (US); Spencer, Tom, Winston-Salem, NC 27103 (US); Wagner, Belinda J., Winston-Salem, NC 27103 (US); Jayo, Manuel J., Winston-Salem, NC 27104 (US); Bertram, Timothy A., Winston-Salem, NC 27104 (US); Ilagan, Roger M., Burlington, NC 27215 (US); Kelley, Russell W., Winston-Salem, NC 27104 (US); Rapoport, H. Scott, 48010 Bilbao Bizkala (ES); Bruce, Andrew, Lexington, NC 27295 (US)
(74) Representative: Vossius & Partner Patentanwälte Rechtsanwälte mbB

(57) **Abstract**

The present invention concerns a non-biased, combinatorial approach to the identification of components that modulate a regenerative response in a target tissue, thereby restoring or partially restoring homeostasis to the target tissue. The methods of the present invention are based on in vivo testing, with or without prior in vitro predictive functional testing or combinatorial testing.

## Description

### Field of the Invention

The present invention concerns a non-biased, combinatorial approach to the identification of components that modulate a regenerative response in a target tissue, thereby restoring or partially restoring homeostasis to the target tissue. The methods of the present invention are based on *in vivo* testing, with or without prior *in vitro* predictive functional testing or combinatorial testing.

### Background of the Invention

The field of regenerative medicine has evolved with a goal of addressing complex degenerative diseases. Several features of regenerative products have emerged as highly relevant to product development:
(1) Following *in vivo* delivery, a regenerative product provides partial or complete homeostasis,
(2) The ultimate function of a regenerative medicine product may be achieved after *in vivo* delivery and integration with the host (i.e., the product provides the necessary impetus, or building blocks, to effect tissue regeneration, but the "building" of the tissue takes place *in situ* through complex temporal and dynamic processes at systemic and microenvironmental levels),
(3) Regenerative products can either provide or recruit the cells and/or cell products (i.e., ECMs, soluble factors, etc.) that help establish the milieu components required to effect the *in situ* restoration of function to the specific tissue or organ.

Regenerative medicine and tissue-engineering technologies frequently include cells and/or biomaterials as components. Some of the features of regenerative products present certain technical challenges relevant to the generation of regenerative product prototypes for testing:
(1) The *in vitro* behavior or characteristics of cells and biomaterials are difficult to measure in *vivo* in most systems. Even when *in vitro* behavior or characteristics can be measured *in vivo,* that behavior rarely correlates to the regenerative outcome
(2) For many applications, a single component (i.e., a single cell type, peptide, protein, or material) has proven insufficient to achieve a robust and durable regenerative outcome.

These challenges suggest that although one of ordinary skill in the art can test the properties of a particular cell, a particular biomaterial, or a particular biomolecule *in vitro,* the *in vivo* regenerative outcome of a single pre-selected component or combinations of pre-selected components based on characteristics defined in *in vitro* testing is unpredictable. However, the majority of technical approaches to developing regenerative products have used trial-and-error methods with components defined by pre-selected characteristics identified *in vitro* to screen for biological effects. A major limitation of this approach is that the characteristics used to pre-select components are few in number compared to the breadth of characteristics that are unknown or uncharacterized.

The strategy of using pre-selected components in trial-and-error screening for regenerative outcome given the aforementioned features of regenerative products and technical challenges renders it difficult if not impossible to relate the cause of any effect observed to the specific characteristics used to pre-select components tested. Several examples from the literature are outlined below.

Despite 20 years of experience working with mesenchymal stem cells (MSC) *in vitro,* no unique marker or functional assay for identifying these cells has been characterized. Fully differentiated cells produce the same results as MSC in some of the differentiation assays that have been used to demonstrate the presence of functional MSC (J Cell Physiol 2007 213:341). Furthermore, unanticipated properties (e.g., excretion of immunosuppressive biomolecules) were observed when MSC were tested *in vivo.* Autologous MSC have been differentiated into specific cell types and combined with biomaterials to effect repair of bone and cartilage with superior morphological and biomechanical properties when compared to control outcomes (J Cell Physiol 2007 213:341), which demonstrates the utility of MSC as an alternative source of tissue-specific cells, not MSC utility for inducing regeneration.

Single cell types have been applied in trial-and-error methodologies to evaluate their regenerative potential in myocardial infarct (J Am Coll Cardiol 2006 47:1777). What has emerged from numerous studies is that distinct cell types (e.g., cardiomyocytes, skeletal myoblasts, smooth muscle cells, fibroblasts, endothelial progenitors, MSC, hematopoietic stem cells, other marrow populations, resident myocardial progenitors, and embryonic stem cells) individually confer equivalent benefit. In fact, one study demonstrated an equivalent effect of MSC transplantation and injection of cell-free supernatant from MSC cultures. The benefit observed (passive mechanical property improvement and amelioration of ventricular remodeling) was not the regenerative outcome which was sought (systolic function restoration), either. These findings demonstrate the insufficiency of single component treatments for achieving regenerative outcomes and highlight the difficulty of elucidating *in vivo* mechanisms of action from trial-and-error methodologies.

Moderate successes have been achieved using autologous or allogeneic sources of the predominant cell type or milieu affected by disease. Primary hepatocytes have been infused into patients with chronic liver disease or inborn errors of hepatic metabolism. Achievement of functional outcomes has been temporary at best; therefore liver transplant remains the standard-of-care (Transplantation 2006 82:441). A widely-used biomaterial matrix constructed from decellularized porcine small intestine submucosa (SIS) marketed by Cook Biomedical has been used to repair diaphragmatic hernias (Chir Ital 2009 61:351) with better clinical outcomes than synthetic mesh, but histochemical analysis of reconstructed partial cystectomies in canines revealed that SIS was ineffective at inducing the regeneration of the muscular layer of the bladder wall (Boruch et al., (2009) J Surg Res, Constructive remodeling of biologic scaffolds is dependent on early exposure to physiologic bladder filling in a canine partial cystectomy model, ePub 20 March 2009). These studies demonstrate that the most obvious first choice of a trial-and-error methodology derived from the disease state may provide short term benefit, but may not yield the regenerative outcome that is sought.

In contrast to trial-and-error methods, a combinatorial method acknowledges the need for multiple components and uses a non-biased system for testing combinations. Furthermore, whole organism contextual data derived from hypothesis-driven testing as well as *in vitro*-generated mechanistic data can be utilized to inform predictions of which combinations may have therapeutic utility. Combinatorial methods that rely on *in vitro* evaluation exist; however, subsequent *in vivo* testing, when it occurs is conducted with a trial-and-error approach. A novel approach involves the use of a combinatorial method for identifying components required to elicit a regenerative response in a target tissue that relies primarily on *in vivo* data to inform decisions for subsequent rounds of testing. One example of a combinatorial method that uses *in vitro* evaluation is that used by the pharmaceutical industry for testing novel compounds for therapeutic biochemical activity (Nat Rev Drug Discov 2005 4:631; Comb Chem High Throughput Screen 2008 11:583; Curr Opin Mol Ther 2000 2:651). Another example is the combinatorial approach used to optimize systems for cell growth or differentiation in culture (http://www.plasticell.co.uk/technology_overview.php; http://www.bd.com/technologies/discovery platform/). Another example is the building of polymer arrays to rapidly screen for cell-polymer interactions in the development of *in vitro* systems for isolating specific cells from heterogeneous populations, differentiating stem cells, and controlling the transfection of cells (Hook et al., (2010) Biomaterials, High throughput methods applied in biomaterial development and discovery, 31(2):187-198, ePub October 7,2009).

In some experimental situations, empirically-generated *in vivo* data can be utilized to inform decisions on subsequent combinatorial testing. *In vivo* experimentation may be used to determine the combinations of cells and biomaterials and delivery methods that form effective regenerative products. Thus, in summary, regenerative outcomes are often driven by factors that cannot be examined through *in vitro* testing (e.g., surprising paracrine effects, interactions with host tissue, etc.) and durable regenerative outcomes are rarely achieved with single-component products (i.e., a single cell type, single bioactive molecule, or a biomaterial-only product).

### Summary of the Invention

In one aspect, the invention concerns a method for identifying inputs necessary to elicit a regenerative response in a target tissue in need of regeneration, comprising
(a) identifying functional elements of a reference tissue of the same type;
(b) identifying functional deficits or abnormalities associated with the target tissue;
(c) isolating the functional elements of the reference tissue, individually or as controlled admixtures;
(d) creating a core list of putative inputs wherein a plurality of functional elements is identified in step (a);
(e) creating a test grid comprising all combinations of putative inputs from the core list; and
(f) conducting *in vivo* experiments based on the test grid created to ascertain the target tissue functional response to the combinations tested; and
(g) based on the results of the *in* vivo experiments, identifying the inputs capable of modulating a regenerative response in the target tissue.

In one embodiment, the reference tissue may be healthy tissue or non-healthy tissue. In a further embodiment, the identified functional deficits or abnormalities associated with the target tissue concern a target tissue in a normal state or in a diseased state.

In a further embodiment, the inputs identified in step (g) may be associated with one or more cell populations. In one embodiment, the cell population(s) are unfractionated or enriched. In another embodiment, the inputs associated with a first cell population may or may not overlap with the inputs associated with a second, third, fourth, and so on, cell population.

In another embodiment, the combination or admixture of two or more enriched cell populations demonstrates an improved regenerative response *in vivo* as compared to a single enriched cell population or an unfractionated cell population.

In another embodiment, the core list of putative inputs created in step (d) is supplemented by additional inputs.

In one other embodiment, the modulation in step (g) concerns inputs capable of eliciting, inputs required to elicit, and/or inputs that contribute to a regenerative response in the target tissue.

In another embodiment, prior to inclusion in the *in vivo* experiments in step (f), one or more putative input is tested individually to determine whether said input has a negative effect on said tissue, or components thereof, where such testing can be *in vivo* or *in vitro.*

In all embodiments, the target tissue of interest may be a target tissue component. In all embodiments, the target tissue may be a target organ. In all embodiments, the target organ of interest may be a target organ component.

In yet another embodiment, if a negative effect is determined during such testing, the input is excluded from the *in vivo* experiments in step (f).

In a further embodiment, if a neutral or positive effect is determined during the testing, the input is included in the *in vivo* experiments in step (f).

In a still further embodiment, the target tissue comprises multiple cellular compartments.

In another embodiment, in step (f) of the method herein function of each cellular compartment is tested.

In yet another embodiment, the method further comprises the step of one or more multivariate analysis of inputs identified.

In a different embodiment, at least some of the inputs identified are cellular components of a regenerative stimulus.

In another embodiment, the cellular components provide direct function *in vivo.*

In yet another embodiment, the cellular components provide indirect stimulation of endogenous elements of the target tissue upon *in vivo* delivery.

In a further embodiment, the cellular components are derived from tissue resident cells.

In another embodiment, the cellular components are derived from cells that are not tissue resident cells. In one embodiment, the non-tissue resident cells are obtained from a source that is not the same source as the target tissue.

In a still further embodiment, the tissue resident cells or non-tissue resident cells are autologous.

In another embodiment, the tissue resident cells or non-tissue resident cells are allogeneic or syngeneic (autogeneic or isogeneic).

In yet another embodiment, the tissue resident cells or non-tissue resident cells are fully differentiated, partially differentiated, or undifferentiated.

In an additional embodiment, at least some of the inputs identified are biomaterials.

In a further embodiment, at least some of the biomaterials facilitate a regenerative response. In one embodiment, the biomaterials provide permissive space in which cells and tissues can form functional structures, and/or for the regenerative stimulus and/or response to occur.

In a still further embodiment, at least some of the biomaterials direct the form or function of cells through natural or engineered biological or biophysical properties.

In a different embodiment, at least some of the inputs identified are bioactive molecules.

In another embodiment, the bioactive molecules comprise cytokines and/or growth factors.

### Brief Description of Figures

Figure 1 is an illustration of biomaterial interactions in regenerative medicine products.
Figure 2 illustrates that 3- and 6-month survival of animals receiving various test articles can be predicted.
Figure 3 illustrates that 3-month survival of animals receiving various test articles can be predicted.
Figure 4 illustrates that 6-month survival of animals receiving various test articles can be predicted.
Figure 5 displays the correlation of each variable (treatment or measurement parameter) with survival.
Figure 6A displays the coefficient plot providing another assessment of positive effects on survival, with consideration of each input or combination of inputs independently.
Figure 6B shows the treatment groups and dose administration for a study conducted in a CKD model.
Figures 6C-D show sCREAT and BUN values in test animals compared to control animals.
Figure 7A-B shows *in vivo* evaluation of biomaterials at 1 week post-implantation.
Figure7C shows *in vivo* evaluation of biomaterials at 4 weeks post-implantation.
Figure 8A-D shows live/dead staining of NKA constructs seeded with canine UNFX cells.
Figure 9A-C shows transcriptomic profiling of NKA constructs.
Figure 10A-B shows the secretomic profiling ofNKA Constructs.
Figure 11A-B shows proteomic profiling of NKA Constructs.
Figure 12A-C shows confocal microscopy of NKA Constructs.
Figure 13A-B shows *in vivo* evaluation of NKA Constructs at 1 and 4 weeks post-implantation.
Figure 14A-D shows *in vivo* evaluation of NKA Construct at 8 weeks post-implantation.

### Detailed Description of the Invention

### I. Definitions

Unless defined otherwise, technical and scientific terms used herein have the same meaning as commonly understood by one of ordinary skill in the art to which this invention belongs.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

Other relevant information is available from text books in the field of tissue engineering, such as, for example, Palsson, Bernhard O., Tissue Engineering, Prentice Hall, 2004 and Principles of Tissue Engineering, 3rd Ed. (Edited by R Lanza, R Langer, & J Vacanti), 2007.

The term "tissue" as used herein refers to a group or collection of similar cells and their intercellular matrix that act together in the performance of a particular function. The primary tissues are epithelial, connective (including blood), skeletal, muscular, glandular and nervous. The term "tissue" specifically includes tissues organized into organs.

The term "cell" or "cells" as used herein refers to any cell population of a tissue.

The term "cell population" as used herein refers to a number of cell obtained by isolation directly from a suitable tissue source, usually from a mammal. The isolated cell population may be subsequently cultured *in vitro.* Those of ordinary skill in the art will appreciate that various methods for isolating and culturing cell populations for use with the present invention and various numbers of cells in a cell population that are suitable for use in the present invention. A cell population may be an unfractionated, heterogeneous cell population dervived from the kidney. For example, a heterogeneous cell population may be isolated from a kidney biopsy or from whole kidney tissue. Alternatively, the heterogeneous cell population may be derived from *in vitro* cultures of mammalian cells, established from kidney biopsies or whole kidney tissue. An unfractionated heterogeneous cell population may also be referred to as a non-enriched cell population.

An "enriched" cell population or preparation refers to a cell population derived from a starting cell population (e.g., an unfractionated, heterogeneous cell population) that contains a greater percentage of a specific cell type than the percentage of that cell type in the starting population. For example, a starting kidney cell population can be enriched for a first, a second, a third, a fourth, a fifth, and so on, cell population of interest. As used herein, the terms "cell population" and "cell preparation" are used interchangeably. The term "cell prototype" may refer to a cell population or a cell population plus a biomaterial.

The term "admixture" as used herein refers to a combination of two or more isolated, enriched cell populations derived from an unfractionated, heterogeneous cell population.

The term "biomaterial" as used here refers to a natural or synthetic biocompatible material that is suitable for introduction into living tissue. A natural biomaterial is a material that is made by a living system. Synthetic biomaterials are materials which are not made by a living system. The biomaterials disclosed herein may be a combination of natural and synthetic biocompatible materials. As used herein, biomaterials include, for example, polymeric matrices and scaffolds. Those of ordinary skill in the art will appreciate that the biomaterial(s) may be configured in various forms, for example, as liquid hydorgel supspensions, porous foam, and may comprise one or more natural or synthetic biocompatible materials.

The term "scaffold" as used herein refers to the configuration of a biomaterial such that it provides a porous space (e.g., rigid, soft, or semi-soft) suitable for the deposition, entrapment, embedding, or attachment of mammalian cells or combinations of cells.

The term "milieu" as used herein refers to the environment that exists within a tissue, comprising the resident cells, non-resident transiently-present cells (such as those of the circulatory system - blood and lymph), the extracellular matrix and other proteins secreted by the cells and accumulated in the tissue, and the peptides, proteins, cytokines, growth factors, salts, and minerals comprising the interstitial fluid and spaces, and the pH, osmolality, oxygen tension, and various gradients thereof established by the architecture and composition of the tissue.

### II. Detailed Description

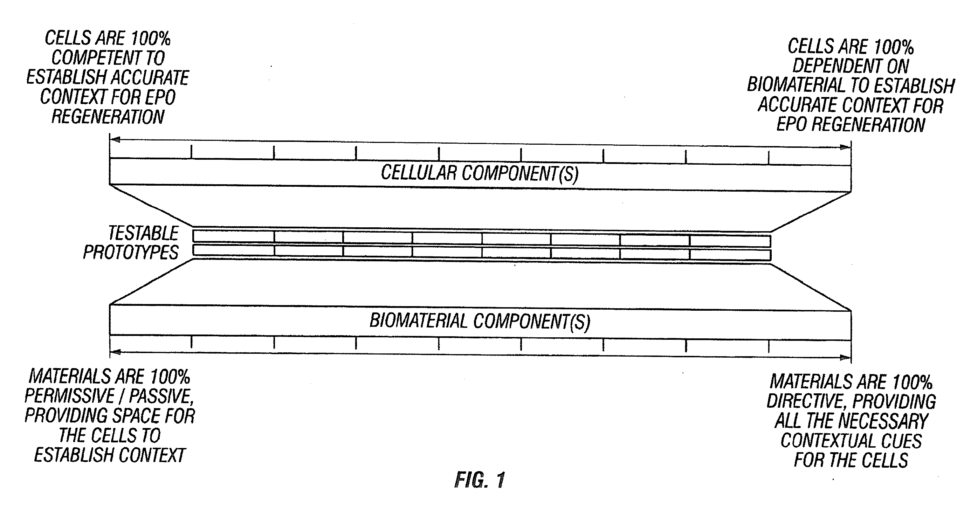

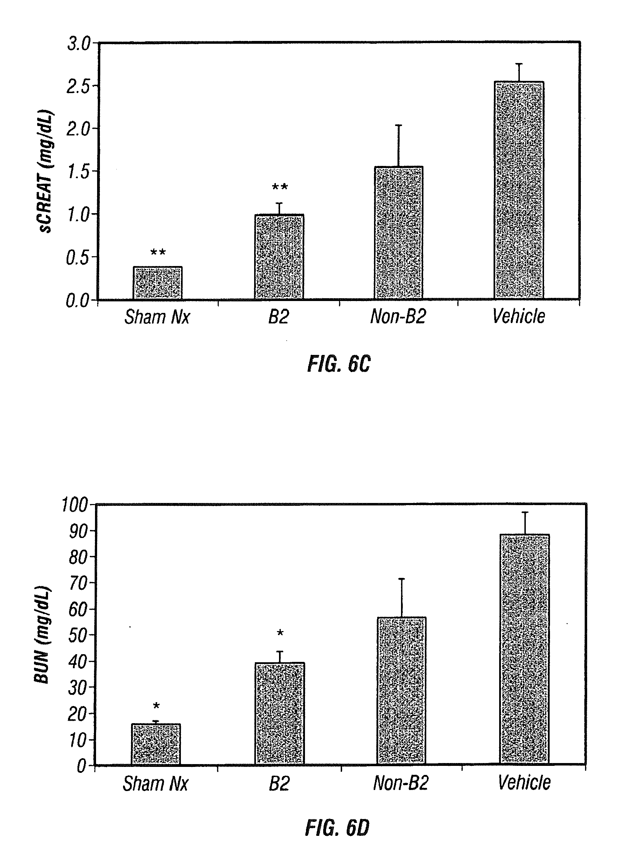

**1. Cells as inputs.** Most tissues in the body are comprised of multiple cell types. The liver, for example, is comprised predominantly of parenchymal hepatocytes (of which there are subtypes), but also contains sinusoidal endothelial cells, bile duct cells, connective tissue cells, and endothelial cells (Cancer Res 1959 19:757). Although the healthy liver has a robust regenerative (or compensatory hyperplasia) response to injury that is driven primarily by the replication of mature hepatocytes (Am. J. Pathol. 1999 155:2135), in circumstances of parenchymal injury due to toxicants or chronic disease such as alcoholic cirrhosis, resident facultative stem cell compartments can also participate in tissue repair and regeneration (Liver 2001 21:367). As another example, the kidney is comprised of glomerular parietal cells, glomerular podocytes, proximal tubular cells, loop of henle cells, distal tubular cells, collecting duct cells, interstitial cells, endocrine cells, endothelial cells, and many highly specialized subtypes and putative resident progenitors (from, The kidney: from normal development to congenital disease, by Vize, Woolf, and Bard). As studies have shown, when renal mass is removed in a healthy animal, the remaining mass (nephrons) undergoes hypertrophy as a form of compensation, involving all cellular compartments of the kidney (Metabolism 2001 50:1418). With regard to cells as 'inputs' for a regenerative product, and in the context of this invention, the following principles are considered:
   i. Cellular component(s) of a regenerative stimulus may provide direct function *in vivo,* or indirect stimulation of endogeneous elements (other cells, extracellular milieu, structure) after *in vivo* delivery.
   ii. Cellular component(s) of a regenerative stimulus may be derived from tissue-resident cells (autologous or allogeneic; fully-differentiated, partially-differentiated, or undifferentiated). Partially differentiated or undifferentiated cells may be meaningful components of a regenerative medicine stimulus whether presented in their undifferentiated form or having been subjected to partial or complete directed differentiation protocols *in vitro* prior to their use.
   iii. These cellular components may be tested *in vitro* for interactions (additive, synergistic, or antagonistic) prior to *in vivo* testing, or the interactions (additive, synergistic, or antagonistic) may be examined solely through *in vivo* testing. The exclusive utilization of *in vitro* testing for interactions (additive, synergistic, or antagonistic) is *not* contemplated as part of this invention.
   According to the methods of the present invention, various cell fractions and/or cell populations may be analyzed *in vivo* to determine whether they contribute to and/or are necessary to elicit a regenerative response in a target tissue in need of regeneration. As described in Example 1, various cell fractions/cell populations were analyzed alone or in combination with biomaterials *in vivo.* Figure 6C-D illustrates how different cell fractions can be identified as contributing to and/or being necessary for eliciting a regenerative response. The B2 fraction was determined to provide superior improvements in sCREAT and BUN as compared to a non-B2 fraction (B3 + B4) and a control.
**2. Materials as inputs:** Biomaterials have been utilized historically in tissue engineering and regenerative medicine approaches, based on both physical and biological attributes (Tuzlakoglu (2009) Tissue Eng Part B Rev 2009 15(1):17-27). Some materials are selected for their ability to provide a permissive space in which cells and tissues can form functional structures, while others are pursued for their ability to potentially direct the form and function of cells through their natural or engineered biological / biophysical properties. With respect to this invention, material components can be considered by the following principles:
   i. The biomaterial component(s) may be comprised of synthetic or naturally-occurring (purified or partially-purified) proteins, peptides, or molecules. The naturally-occurring biomaterial components may be produced by one or more cellular component(s), either before implantation or *in situ,* after implantation.
   ii. The biomaterial component(s) may be utilized in their base form or modified to present a specific structure or function, by the passive or active coupling of bioactive components (such as cytokines, growth factors, inhibitors, or pharmacological agents be they naturally-occurring or synthesized).
   iii. The biomaterial component(s) may be presented in a range of physical forms, including but not limited to rigid porous scaffolds, soft porous scaffolds, hydrogels of varying density and concentration, or liquids, or as a matrix produced by administered cells.
   iv. The biomaterial component(s) may be used as single agents or in combination with other biomaterial components, and any combinations utilized need not employ biomaterials presented in the same form (solid, hydrogel, or liquid).
   According to the methods of the present invention, biomaterial components may be analyzed *in vivo* to determine whether they contribute to and/or are necessary to elicit a regenerative response in a target tissue in need of regeneration. As described in Examples 1 and 2, various biomaterials were analyzed alone or in combination with kidney cells *in vivo.* After implantation of a hydrogel-based NeoKidney Augment (NKA) construct, evidence of regeneration in the kidney was observed.
**3. Bioactive molecules as inputs:** The use of pharmacological agents, cytokines, and/or growth factors as adjuncts to tissue-engineered or regenerative products has been contemplated and put into practice. Perhaps one of the most data-backed examples of this approach has been the passive or active coupling of vEGF (vascular endothelial growth factor) to materials prior to implant to facilitate vascularization of the implant (Curr Stem Cell Res Ther 2006 1:333). With respect to this invention, bioactive molecules (including drugs, cytokines, growth factors, peptides, proteins, or chemical moieties) could be included as inputs, presented in solid, liquid, or gel form. These molecules could be introduced as independent inputs or coupled directly to cellular or biomaterial inputs using either active or passive coating / coupling procedures. With respect to this invention, these molecules are considered by the following principles:
   i. The bioactive molecule component(s) may consist of novel or existing pharmaceutical compounds. It is noted that many biomaterials are in fact bioactive as well.
**4. Cell:Biomaterial interactions in regenerative medicine products:** As discussed preliminarily in sections 1 & 2 above, cellular components of a regenerative medicine product potentially may be contemplated and delivered as fully functional (e.g., 100%) and competent to deliver the needed regenerative outcome without the addition of other components. In related scenarios, the material components may be absent or relatively inert, providing only permissive space for the cells to function. In these scenarios, one or more cell types might be required to achieve the desired outcome, and these cellular components could consist of any source or state of cell contemplated in section 1 above. In the other extreme, the regenerative stimulus may be delivered by a material that is potentially 100% competent of achieving a regenerative outcome without the use of cells or other bioactive molecules as part of the product. In a related scenario, cells are delivered but are completely or partially dependent on the material component to direct the outcome. As depicted in representative Figure 1, all possibilities between these two extremes exist across the spectrum of providing cell-only or material-only regenerative products.
**5. General description of the invention** The invention described herein may be defined as the application of a non-biased, combinatorial approach to the identification and optimization of the components required to elicit a regenerative response in a target tissue, thereby restoring or partially restoring homeostasis to that tissue, as determined by *in vivo testing* either with or without prior *in vitro* predictive functional testing or combinatorial testing.
**6. General scheme for application of the invention:**
   a. Identify target organ / tissue for regenerative product
      i. Identify the known cellular, compositional, and/or structural deficits or abnormalities associated with degeneration of that target organ/tissue
      ii. Identify the known cellular and other (i.e., ECM, soluble factors, etc.) components of that healthy target organ / tissue.
   b. Develop list of putative inputs based on components (partial or complete) of healthy tissue
      i. One approach would insure that each functional element of a healthy tissue was represented in the list of putative inputs (= **core list);** this could occur by testing the individual elements individually or as controlled admixtures.
      ii. Optionally, supplement the core **list** with additional inputs, which may include synthetic or naturally-occurring biomaterials, or any other cellular, material, or bioactive molecule input as described in sections 1-3.
   c. Utilize manual or automated methods to generate test grids that contemplate all possible combinations (this can be done in a variety of ways, including full factorial or fractional factorial designs, as described in detail in Experimental Design and Data Analysis for Biologists by Gerald Peter Quinn & Michael J. Keough; also taught *in* Experimental Design in Biotechnology by Perry D. Haaland).
   d. Using the test grids as a guide, conduct the *in vivo* experiments necessary to ascertain regenerative outcome and/or function of the various combinations. If positive or negative controls exist pertinent to the model, these should be included in every series of experiments.
      i. *Optional*: Prior to testing combinations, each component may be tested individually to determine whether any overt negative effects are associated with that component. This testing could occur either *in vivo,* or *in vitro,* providing there are assays available that would detect potential toxic effects toward the target tissue. The results *may* prompt user to exclude such component(s) from the screening. However, all components that yield positive or neutral results in an individual screen should ideally be included in the *in vivo* combination testing. Indeed, the method can be viewed as a self-modifying or adaptive algorithm that continually updates itself as new information on its elements is obtained.
      ii. *Optional:* While it is preferred that all possible combinations be tested, it is recognized that experimental and/or logistical limitations may impose restrictions. Thus, it is possible to test a subset of combinations contemplated by the grid, or to conduct the experiments in series or batches. If the 'batch' approach is taken, it is important that each batch contain the same positive and negative control treatments so that data can be normalized between runs. In the case that testing all possible combinations is too cumbersome or expensive, the fractional factorial approach would be preferred over taking the full factorial approach but only testing a handful of all possible combinations. Other approaches include, for example, variations on deconvolution analyses, i.e.. breaking a large data set apart, analyzing individual clusters and reassembling the data. Interrelationships among clusters are preserved upon reassembly of the large data block.
   e. Collect data from the *in vivo* experiments that enable regenerative outcome(s) to be captured via direct or indirect methods. It is preferred that more than one measurement be taken. In the context of the target tissue being a fairly complex tissue (consisting of multiple cellular compartments as well as certain biophysical and biochemical properties), that at least one measurement be taken to assess function of each of the cellular compartments. Ideally, the biophysical and biochemical properties of the organ / tissue are assessed +/- various treatments (i.e., size, weight, density, and relevant biomechanical properties)
   f. Conduct multivariable analyses that consider:
      i. Each component input
      ii. Each output parameter measurement
      iii. Interactions among components (if possible to detect)
   g. Utilize resulting data to select prototype, or use resulting data to design new combinatorial experiments based on outcomes; this can be applied to optimize for specific regenerative features or to build custom products for specific disease states in a target tissue (for example, some diseases of the kidney, such as acute tubular necrosis, may require tubular regeneration, while other diseases of the kidney, such as glomerulosclerosis, may require regeneration of glomerular components).

The subject matter of the present application is related to U.S. Provisional Application Nos. 61/114,025 filed November 12, 2008, 61/114,030 filed November 12, 2008, 61/201,056 filed December 5, 2008, 61/201,305 filed December 8, 2008, 61/121,311 filed December 10, 2008, and U.S. Application No. 12/617,721 filed on November 12, 2009, the disclosures of which are incorporated herein by reference.

All references cited herein are incorporated herein by reference in their entireties. Further details of the invention are illustrated by the following non-limiting examples.

### Example 1- General Protocol for a Combinatorial Approach to the Kidney

Chronic kidney disease (CKD) is a progressive disease that ultimately leads to severe organ degeneration and failure, requiring dialysis or whole organ transplant. As such, there is a need to derive a regenerative medicine product specifically for the purpose of stabilizing, repairing, and/or regenerating a chronically-diseased kidney. As the kidney is a complex organ containing a large number of function-specific cell types, the pathogenesis of CKD involves multiple cellular (parenchymal) and hypocellular (stroma) compartments. The tubular cell compartment is compromised as evidenced by disease characterized by tubular degeneration, atrophy, luminal dilatation with cellular debris and proteinaceous casts, and the development of tubulo-interstitial fibrosis. The glomerular compartment is compromised, as evidenced by glomerular hypertrophy, atrophy, and sclerosis. The functional aspects of the endocrine compartment(s) of the kidney are compromised, as evidenced by the epo-deficiency and anemia of CKD, vitamin D deficiencies, and disturbances in the renin-angiotensin system leading to hypertension. The vascular compartment is compromised, as evidenced by hypertension, altered tubular-glomerular-feedback mechanisms, and inflammatory aggregates with interstitial fibrosis. The collecting duct system is compromised, as evidenced by interstitial fibrosis, and the epithelial-mesenchymal-transformation of collecting duct epithelium. The systemic evidence of: 1) proteinuria and albuminuria support both the glomerular and tubular disease; 2) hypercholesterolemia provides clear evidence of glomerular disease; and 3) increased serum/plasma levels of blood urea nitrogen and creatinine provide additional evidence of glomerular disease. The cellular and other related aspects of the healthy adult kidney are tubular cells (proximal); tubular cells (distal); collecting duct cells; vascular cells (afferent / efferent arterioles, endothelial cells, vascular smooth muscle cells); erythropoietin-producing interstitial fibroblasts; other interstitial cells; resident progenitors (various); glomerular cells (podocytes, mesangial, endothelial); Specific regional architecture (cortical, cortico-medullary, medullary, calyx); Ionic and oxygen gradients (spatial); Tubular basement membrane (laminin, collagen IV, perlecan); Glomerular basement membrane (collagen IV, laminin, nidogen, and heparin sulfate proteoglycans); and renal pelvis. A list of the putative inputs based on components (partial or complete) of healthy tissue is provided below.
i. CORE LIST:
   1. tubular cells (proximal)
   2. tubular cells (distal)
   3. collecting duct cells
   4. epo-producing cells
   5. glomerular cells
   6. vascular cells
   7. resident progenitor populations (various)
ii. ADDITIONAL INPUTS:
   1. Hyaluronic acid (due to its expression by certain cells of the kidney and during development)
   2. Synthetic biomaterial candidates (PLA-based, selected based on *in vitro* screening)
   3. Collagen (various forms)

Standard methods were employed to culture heterogeneous mixtures of cells from whole kidney tissue or biopsied kidney tissue. These heterogeneous, or unfractionated (UNFX) cells were isolated successfully from normal and diseased tissues from rat, dog, swine, and human. Cultured UNFX cells were confirmed to contain cells from all major compartments of the kidney (collecting duct, tubular, vascular, glomerular, endocrine, interstitial, and resident progenitor). See Example 18 of U.S. Application No. 12/617,721 filed on November 12, 2009. Cellular compartments were separated conveniently through the use of a density step gradient optimized for kidney (see Example 8 of U.S. Application No. 12/617,721 filed on November 12, 2009), using Optiprep (iodixanol) density gradient media. Individual fractions containing enriched proportions of specific cells on the CORE LIST were characterized by gene expression and functional attributes. Two individual fractions were tested alone (tubular cells w/ some collecting duct cells, a.k.a. B2) and a rare subpopulation containing an admixture of glomerular cells, erythropoietin-producing cells, and vascular cells (a.k.a., B4). See Example 10 of U.S. Application No. 12/617,721 filed on November 12, 2009. These fractions were selected based on *in vitro* attributes and hypothetical involvement in repair / regeneration in the tubular and endocrine compartment, respectively. Surprisingly, the B2 subpopulation, which contains tubular cells but is depleted of epo-producing cells, was highly effective at restoring erythroid homeostasis - a feature that would be hypothetically-associated with B4 (not B2). Thus, it followed that more fractions (compartments) and combinations thereof should be tested so that unexpected effects (singular, synergistic, or additive) could be tested. Thus cell:material and cell:cell combinations were tested as illustrated in the following grid:

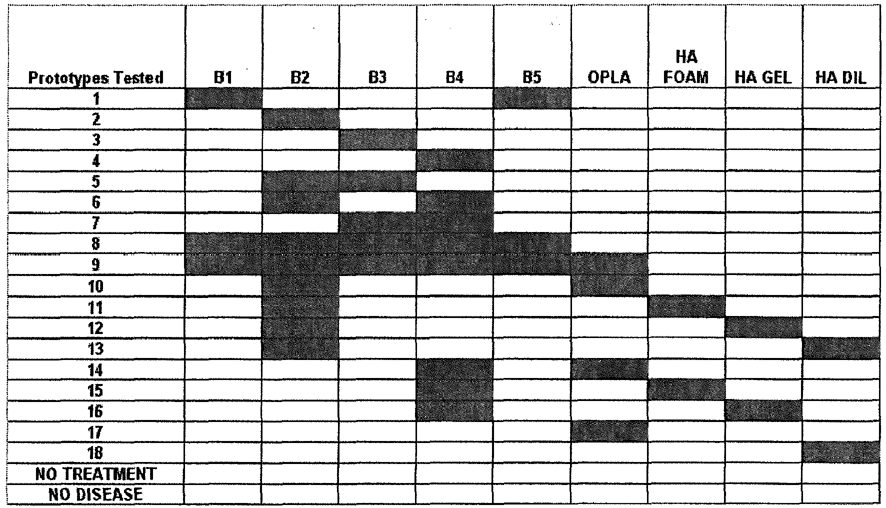

B1-B5 refer to enriched cell populations obtained from the kidney. B2 is comprised predominantly of tubular cells, containing mostly proximal tubular cells capable of robust albumin uptake, with some distal tubule and collecting duct cells present. Other confirmed cell types (endocrine, glomerular, vascular) are present only in trace quantities; B4 is comprised of endocrine, vascular, and glomerular cells, but including also some small tubular cells, predominantly proximal in nature. B1 is comprised predominantly of distal tubular and collecting duct cells, with trace amounts of other cell types present. B3 is comprised predominantly of proximal tubular cells, with a small quantity of endocrine, vascular, and glomerular cells. B5 is comprised of very small cells, endocrine, vascular, and progenitor-like in nature; this fraction also contains cells with low viability, and represents a very small portion of the population overall. OPLA refers to an open-cell polylactic acid (OPLA®). HA FOAM refers to hyaluronic acid in porous foam form. HA GEL refers to hyaluronic acid (HA) in hydrogel form. HA DIL refers to hyaluronic acid in liquid form.

*In vivo* experiments were conducted to compare the effects of the above cell, cell/cell combinations, and cell/biomaterials in stimulating a regenerative outcome when introduced intra-renally, after disease onset in a terminal model of CKD. A wide range of systemic functional parameters were evaluated to examine evidence of repair and/or regeneration across all cellular compartments; these parameters included erythropoiesis (hematocrit, red blood cell number), renal filtration (blood urea nitrogen, serum creatinine), calcium-phosphorous balance (serum calcium, phosphorous), lipid metabolism (cholesterol, triglycerides), and protein retention (serum protein, urinary protein). See Examples 14 and 15 of U.S. Application No. 12/617,721 filed on November 12, 2009. Systemic and histologic data were collected from the combinatorial experiments. Data were collected for six months post-treatment and accumulated into spreadsheets for subsequent analysis (see Table below).

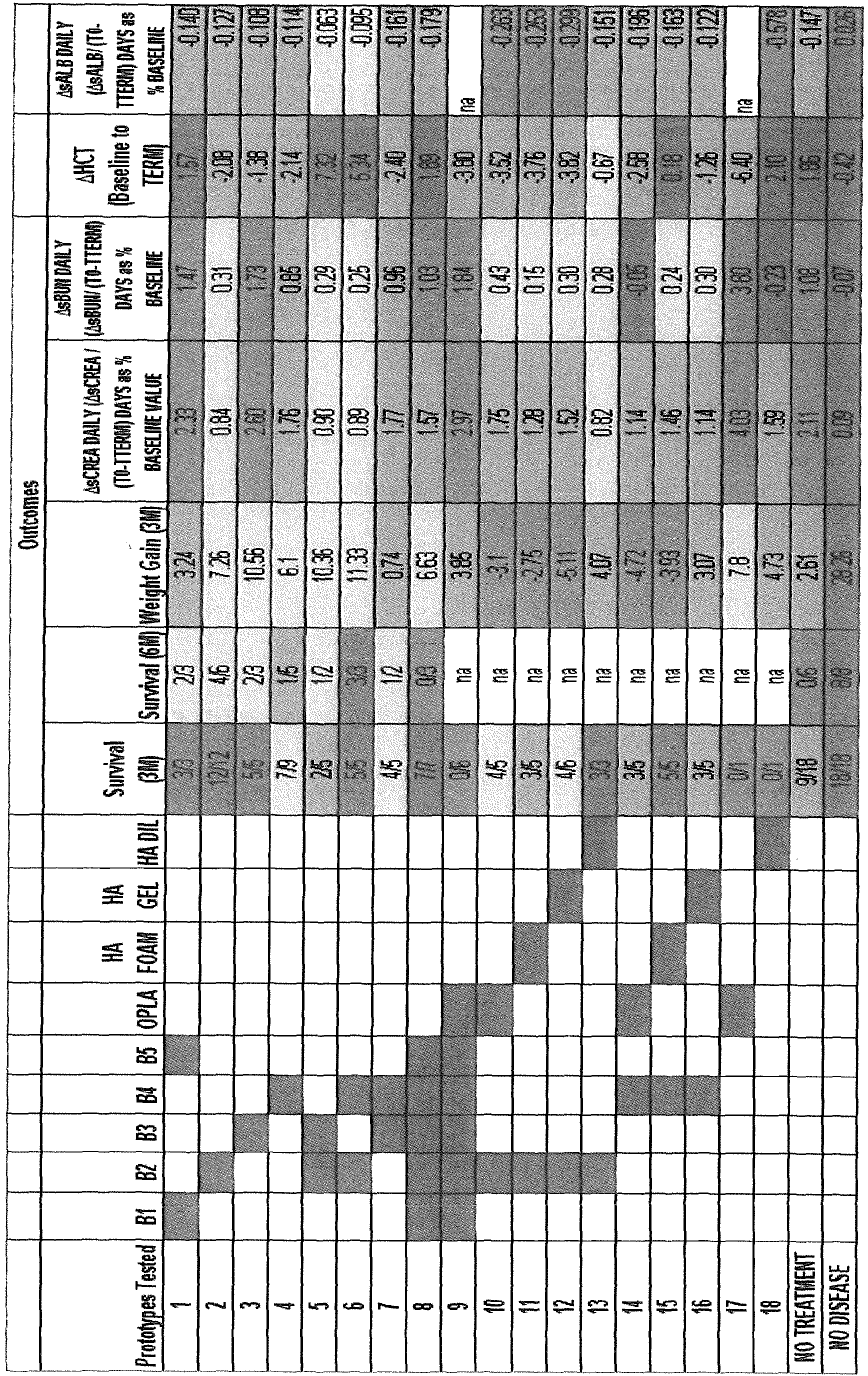

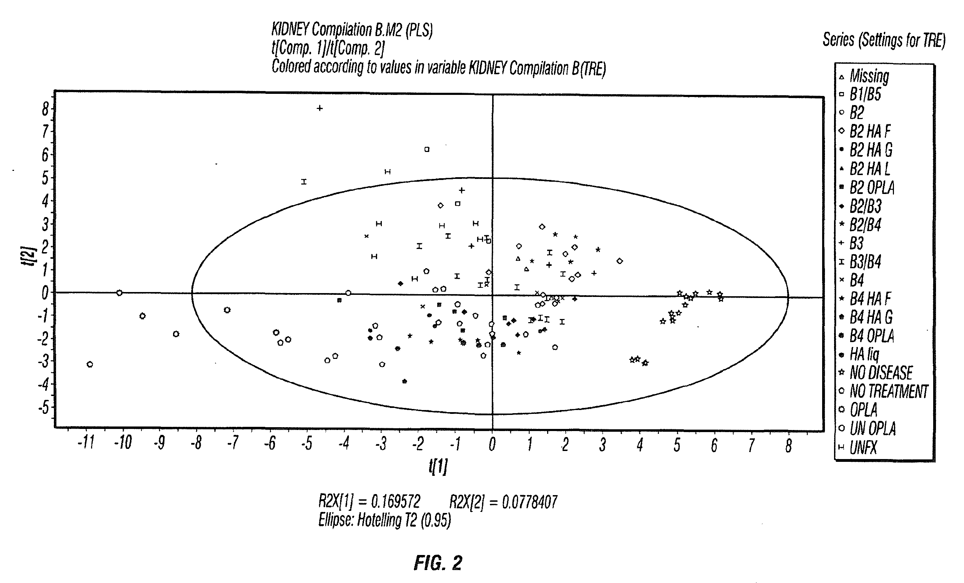

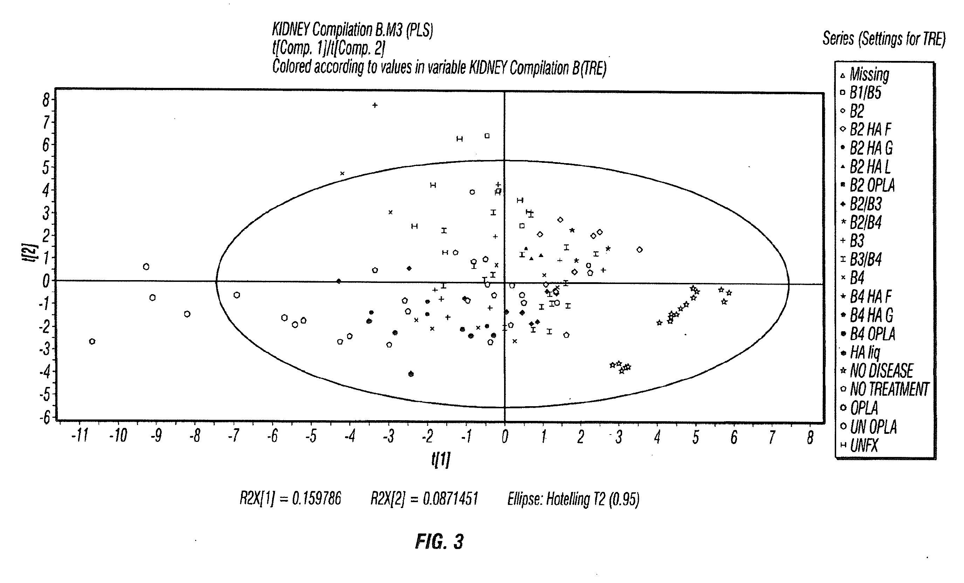

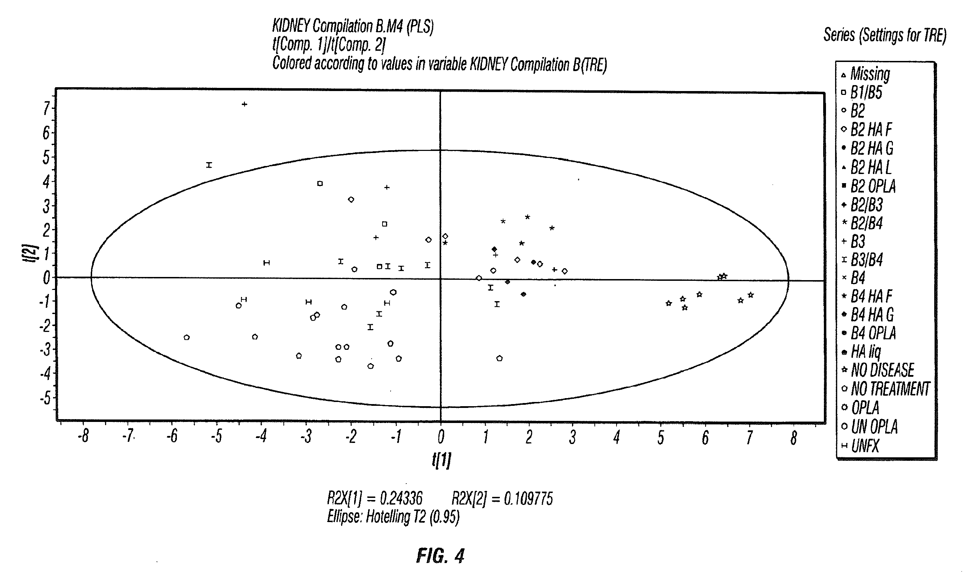

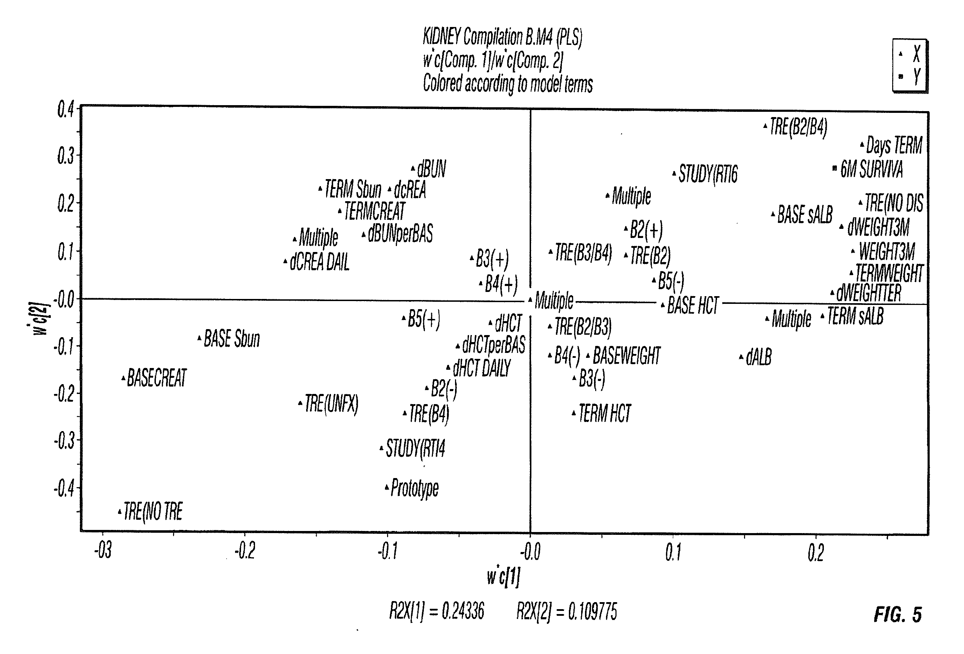

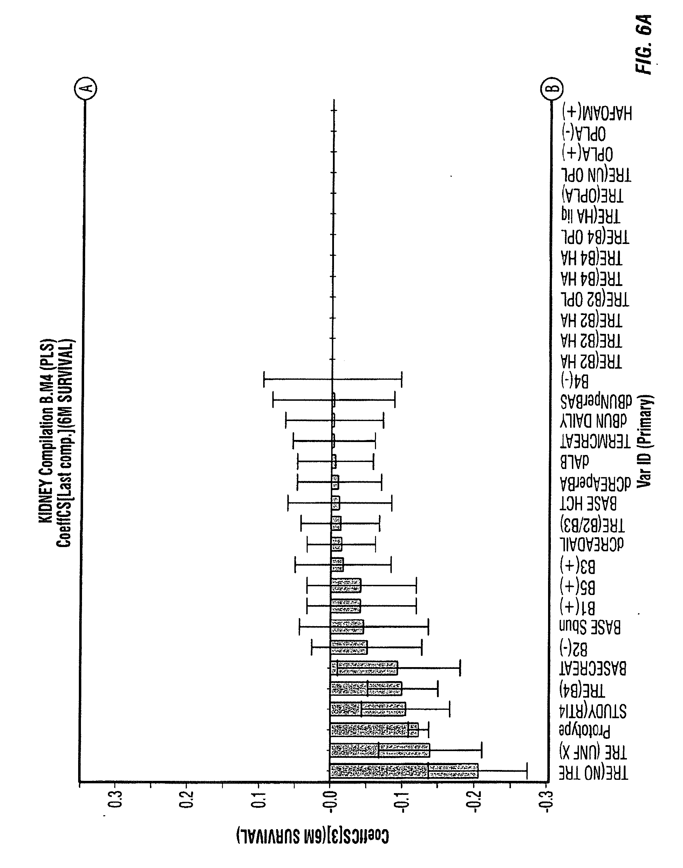

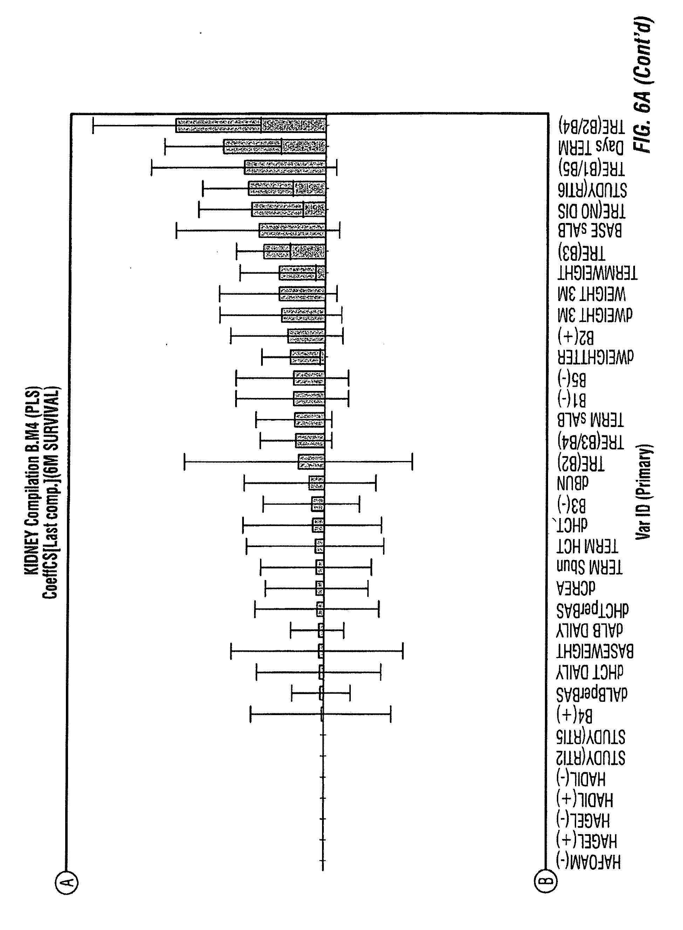

All data were subjected to multivariable analyses. Analyses included consideration of individual inputs, multivariable output parameters, and interactions among input components. Multivariate analysis was performed on data generated from the *in vivo* testing of 18 cell, cell/cell, cell/biomaterial prototypes of NeoKidney Augment (NKA). The following plots show that 3- and 6-month survival (Figure 2), 3-month survival (Figure 3), and 6-month survival (Figure 4) can be predicted by having delivered specific treatments. Prototypes containing 'B2' provided strong support for survival at both the 3- and 6-month timepoints post-treatment. Non-diseased and Diseased/No Treatment rats clustered together and in different quadrants of the analysis, supporting the study observations that Non-diseased rats were healthy and had 100% survival, while Diseased/No Treatment rats developed progressive disease and had 100% death within the timeframe(s) of the study (up to six months). Figure 5 displays the correlation of each variable (treatment or measurement parameter) with survival, and again highlights the strength of the model overall. Interestingly, the strongest correlates with 6-month survival in this analysis (besides survival days) were treatment with B2 or B2/B4 (see upper right quadrant of Figure 5). The coefficient plot (Figure 6A) provides another assessment of positive effects on survival, highlighting positive effectors such as body weight, baseline serum Albumin, treatment with presence of B2, the B2/B4 combination prototype, the presence of B4, the absence of B1, absence of B5, absence of B3. In contrast, poor survival was correlated with NO TREATMENT, treatment with UNFX, the presence of B1, the presence of B5, the presence of B3, high baseline creatinine, and high baseline BUN. In summary, these analyses across (4) rodent studies support continued investigation into prototypes consisting of B2 +/- B4 component.

The resulting data provided evidence for the selection of B2+B4 as a favorable prototype for continued studies. Additional combinatorial studies will utilize these data as a foundation upon which to design an optimization experiment to better define the product prototype and required components for achieving optimal regenerative outcome *in vivo.*

In another experiment, different cell fractions obtained by the methods described above were tested *in vivo* in a 5/6 nephrectomized (two-step) CKD model. Female Lewis rats were obtained from Charles River Laboratories. Rats were anesthetized and remnant kidneys were exposed via ventral medial-lateral incision. Cells were suspended in 100µL sterile PBS, loaded into a 1cc syringe fitted with a ½ inch 23G needle (Becton Dickinson), and delivered directly to the kidney through the apical cortex at a depth of approximately 3-5mm. In Studies A and B cells were delivered to rats 6-12 hours after cell harvest.

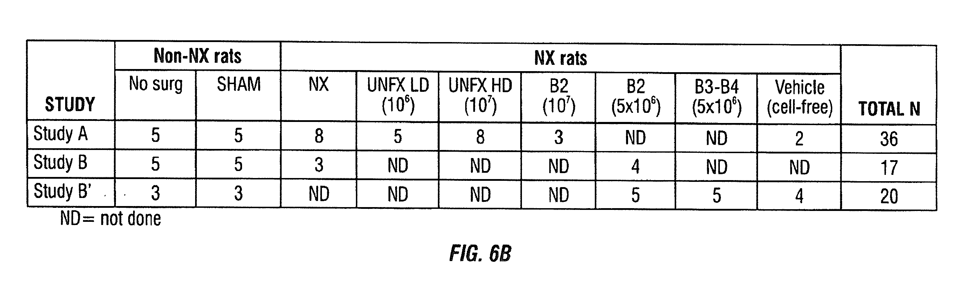

Figure 6B displays the treatment groups and dose administration in Studies A and B. In Study B', the following treatment groups were appended: 1) additional B2 rats (n=5), delivered at (5 x 10⁶) generated from an independent preparation of UNFX; 2) non-B2 cells (n=5), delivered at (5 x 10⁶), isolated from the same UNFX preparation, with the non-B2 cell mixture generated by combining two of the adjacent bands (B3 and B4) produced by the density gradient centrifugation protocol; 3) cell-free Vehicle controls (n=4), comprising injection diluent (sterile PBS). In Study B', all treatments were delivered 18-24 hours after cell harvest to better approximate a feasible clinical scenario (i.e., a timeframe compatible with overnight shipment).

The *in vivo* bioactivity of UNFX and B2 in CKD rodents was analyzed. Two iterative studies were conducted to evaluate the efficacy of orthotopic delivery of UNFX and/or B2 cells to remnant kidneys of NX rats (Figure 6B-D). Treatment was initiated after progressive renal failure was established (persistent >200% elevation in sCREAT and >150% elevation in BUN). In Study A, the heterogeneous UNFX cell population was delivered at high (107) and low (106) doses and compared with a high dose (107) of B2, untreated NX and healthy Sham NX rats. Low dose UNFX had a mild but transient survival benefit at 12 weeks, or 90 days (data not shown), but neither dose of UNFX significantly reduced the severity of disease present in the NX rats (data not shown). In contrast to UNFX, treatment with B2 extended survival beyond the 90 day time point through study completion at 6 months, or 180 days.

Figure 6C-D shows significant improvement in systemic parameters associated with filtration function (sCREAT and BUN). Improvements were also observed in protein handling (sALB and A:G ratio), and general health (body weight) (data not shown). Mild trends of improvement in erythropoiesis (HCT and HB) and mineral balance (sPHOS) were also noted with B2 treatment, but did not reach statistical significance at the 12-week time point.

Study B was designed to confirm the in vivo effectiveness of B2 observed in Study A in an independent experiment. A more physiologically-relevant dose of B2 (5 x 10⁶) was administered in Study B to reduce the volume delivered into the remnant kidneys. B2 treatment in Study B resulted in 100% survival (data not shown) at 12 weeks (90 days) and had stabilizing effects on sCREAT and sBUN (data not shown); nearly identical to those observed in Study A. In contrast 0% of NX rats survived 90 days. While trends of improvement were noted in other systemic parameters after B2 treatment in Study B (e.g., sALB, sPHOS), statistical significance was not achieved (data not shown). Finally, the Study B design was modified (Study B') to compare the observed systemic effects of B2 on renal filtration function to cell-free Vehicle controls and to treatment with an equivalent dose (5 x 10⁶) of non-B2 cells derived from the same UNFX starting population after density gradient separation.

Figure 6C-D shows healthy Sham NX rats and B2 rats exhibited significantly lower sCREAT and BUN values compared to cell-free Vehicle controls at 12 weeks post-treatment. While the non-B2 rats trended towards improvement in sCREAT and BUN, they remained statistically undifferentiated from Vehicle controls. Consistent with the outcomes observed in Studies A & B, the B2 group was characterized by 100% (5/5) survival 12 weeks post-implant, compared to 60% (3/5) for the non-B2 group and 50% (2/4) for the Vehicle group.

Additional details related to this study can be found in Kelley *et al.* Am J Physiol Renal Physiol 2010 Nov;299(5):F1026-39. Epub 2010 Sep 8.

### Example 2 - Functional evaluation of Neo-Kidney Augment Constructs

Renal cell populations seeded onto gelatin or HA-based hydrogels were viable and maintained a tubular epithelial functional phenotype during an *in vitro* maturation of 3 days as measured by transcriptomic, proteomic, secretomic and confocal immunofluorescence assays.

### Materials and Methods.

**Biomaterials.** Biomaterials were prepared as beads (homogenous, spherical configuration) or as particles (heterogenous population with jagged edges). Gelatin beads (Cultispher S and Cultispher GL) manufactured by Percell Biolytica (Astorp, Sweden) were purchased from Sigma-Aldrich (St. Louis, MO) and Fisher Scientific (Pittsburgh, PA), respectively. Crosslinked HA and HA/gelatin (HyStem™ and Extracel™ from Glycosan BioSystems, Salt Lake City, UT) particles were formed from lyophilized sponges made according to the manufacturer's instructions. Gelatin (Sigma) particles were formed from crosslinked, lyophilized sponges.

PCL was purchased from Sigma-Aldrich (St. Louis, MO). PLGA 50:50 was purchased from Durect Corp. (Pelham, AL). PCL and PLGA beads were prepared using a modified double emulsion (W/O/W) solvent extraction method. PLGA particles were prepared using a solvent casting porogen leaching technique. All beads and particles were between 65 and 355 microns when measured in a dry state.

**Cell isolation, preparation and culture.** Cadaveric human kidneys were procured through National Disease Research Institute (NDRI) in compliance with all NIH guidelines governing the use of human tissues for research purposes. Canine kidneys were procured from a contract research organization (Integra). Rat kidneys (21 day old Lewis) were obtained from Charles River Labs (MI). The preparation of primary renal cell populations (UNFX) and defined sub-populations (B2) from whole rat, canine and human kidney has been previously described (Aboushwareb et al. World J Urol 26(4):295-300; 2008; Kelley *et al.* 2010 *supra*).

Am J Physiol Renal Physiol (September 8, 2010) doi:10.1152/ajprenal.00221.2010; Presnell et al. WO/2010/056328). In brief, kidney tissue was dissociated enzymatically in a buffer containing 4.0 units/mL dispase (Stem Cell Technologies, Inc., Vancouver BC, Canada) and 300 units/ml collagenase IV (Worthington Biochemical, Lakewood NJ), then red blood cells and debris were removed by centrifugation through 15% iodixanol (Optiprep®, Axis Shield, Norton, MA) to yield UNFX. UNFX cells were seeded onto tissue culture treated polystyrene plates (NUNC, Rochester NY) and cultured in 50:50 media, a 1:1 mixture of high glucose DMEM:Keratinocyte Serum Free Medium (KSFM) containing 5% FBS, 2.5µg EGF, 25mg BPE, 1X ITS (insulin/transferrin/sodium selenite medium supplement), and antibiotic/antimycotic (all from Invitrogen, Carlsbad CA). B2 cells were isolated from UNFX cultures by centrifugation through a four-step iodixanol (OptiPrep; 60% w/v in unsupplemented KSFM) density gradient layered specifically for rodent (16%, 13%, 11%, and 7%), canine (16%, 11%, 10%, and 7%), or human (16%, 11%, 9%, and 7%) (Presnell et al. WO/2010/056328; Kelley *et al.* 2010 *supra).* Gradients were centrifuged at 800 x g for 20 minutes at room temperature (without brake). Bands of interest were removed via pipette and washed twice in sterile phosphate buffered saline (PBS).

**Cell/biomaterial composites (NKA Constructs).** For *in vitro* analysis of cell functionality on biomaterials, a uniform layer of biomaterials (prepared as described above) was layered onto one well of a 6-well low attachment plate (Costar #3471, Corning). Human UNFX or B2 cells (2.5 x 10⁵ per well) were seeded directly onto the biomaterial. For studies of adherence of canine cells to biomaterials, 2.5 x 10⁶ UNFX cells were seeded with 50µl packed volume of biomaterials in a non-adherent 24-well plate (Costar #3473, Corning). After 4 hours on a rocking platform, canine NKA Constructs were matured overnight at 37° C in a 5% CO₂ incubator. The next day, live/dead staining was performed using a live/dead staining assay kit (Invitrogen) according to the manufacturer's instructions. Rat NKA Constructs were prepared in a 60 cc syringe on a roller bottle apparatus with a rotational speed of 1 RPM.

For the transcriptomic, secretomic, and proteomic analyses described below, NKA Constructs were matured for 3 days. Cells were then harvested for transcriptomic or proteomic analyses and conditioned media was collected for secretomic profiling.

**Functional analysis of tubular cell associated enzyme activity.** Canine NKA Constructs (10µl loose packed volume) in 24-well plates were evaluated using an assay for leucine aminopeptidase (LAP) activity adapted from a previously published method (Tate et al. Methods Enzymol 113:400-419; 1985). Briefly, 0.5ml of 0.3mM L-leucine p-nitroanalide (Sigma) in PBS was added to NKA Constructs for 1 hour at room temperature. Wells were sampled in duplicate and absorbance at 405nm recorded as a measure of LAP activity. LLC-PK1 cell lysate (American Type Culture Collection, or ATCC) served as the positive control.

**Transcriptomic profiling.** Poly-adenylated RNA was extracted using the RNeasy Plus Mini Kit (Qiagen, CA). Concentration and integrity was determined by UV spectrophotometry. cDNA was generated from 1.4µg isolated RNA using the SuperScript VILO cDNA Synthesis Kit (Invitrogen). Expression levels of target transcripts were examined by quantitative real-time polymerase chain reaction (qRT-PCR) using commercially available primers and probes **(Table 33.1)** and an ABI-Prism 7300 Real Time PCR System (Applied Biosystems, CA). Amplification was performed using TaqMan Gene Expression Master Mix (ABI, Cat# 4369016) and TATA Box Binding Protein gene (TBP) served as the endogenous control. Each reaction consisted of 10µl Master Mix (2X), 1µl Primer and Probe (20X) and 9µl cDNA. Samples were run in triplicate.

**Table 2.1**

| **Human TaqMan Primers/Probes** | | | |
|---|---|---|---|
| **Gene** | **Abbrv.** | **Marker** | **TaqMan Cat#** |
| Aquaporin 2 | AQP2 | Distal Collecting Duct Tubule | Hs00166640_m1 |
| Epithelial Cadherin/Cadherin 1, Type 1 | CDH1/ECAD | Distal Tubule | Hs00170423_m1 |
| Neuronal Cadherin/Cadherin 2, Type 1 | CDH2/NCAD | Proximal Tubule | Hs00169953_m1 |
| Cubilin, Intrinsic Factor-Cobalamin Receptor | CUBN | Proximal Tubule | Hs00153607_m1 |
| Nephrin | NPHS1 | Glomerular/Podocyte | Hs00190466_m1 |
| Podocin | NPHS2 | Glomerular/Podocyte | Hs00922492_m1 |
| Erythropoietin | EPO | Kidney Interstitum | Hs01071097_m1 |
| Cytochrome P450, Family 24, Subfamily A, Polypeptide 1/Vitamin D 24-Hydroxylase | CYP2R1 | Proximal Tubule | Hs01379776_m1 |
| Vascular Endothelial Growth Factor A | VEGFA | Endothelial/Vascular | Hs00900055_m1 |
| Platelet/Endothelial Cell Adhesion Molecule | PECAM1 | Endothelial/Vascular | Hs00169777_m1 |
| Smooth Muscle Myosin Heavy Chain | MYH11/SMMHC | Smooth Muscle | Hs00224610_m1 |
| Calponin | CNN1 | Smooth Muscle | Hs00154543_m1 |
| TATA Box Binding Protein | TBP | Endogenous Control | Hs99999910_m1 |

**Secretomic profiling.** Conditioned medium from human NKA Constructs was collected and frozen at -80° C. Samples were evaluated for biomarker concentration quantitation. The results for a given biomarker concentration in conditioned media were normalized relative to the concentration of the same biomarker in conditioned media from control cultures (2D culture without biomaterial) and expressed as a unitless ratio.

**Proteomic profiling.** Protein from three independent replicates was extracted from cell/biomaterial composites and pooled for analysis by 2D gel electrophoresis. All reagents were from Invitrogen. Isoelectric focusing (IEF) was conducted by adding 30µg of protein resuspended in 200µl of ZOOM 2D protein solubilizer #1 (Cat# ZS10001), ZOOM carrier ampholytes pH 4-7 (Cat# ZM0022), and 2M DTT (Cat# 15508-013) to pH 4-7 ZOOM IEF Strips (Cat# ZM0012). Following electrophoresis for 18 hours at 500V, IEF strips were loaded onto NuPAGE Novex 4-12% Bis-Tris ZOOM IPG well gels (Cat# NP0330BOX) for SDS-PAGE separation and electrophoresed for 45 min at 200V in MES buffer (Cat# NP0002). Proteins were visualized using SYPRO Ruby protein gel stain (Cat# S-12000) according to the manufacturer's instructions.

**Confocal microscopy.** NKA Constructs prepared from human or rat UNFX or B2 cells were matured for 3 days and then fixed in 2% paraformaldehyde for 30 minutes. Fixed NKA Constructs were blocked and permeabilized by incubation in 10% goat serum (Invitrogen) in D-PBS (Invitrogen) + 0.2% Triton X-100 (Sigma) for 1 hour at room temperature (RT). For immunofluorescence, NKA Constructs were labeled with primary antibodies (Table 33.2) at a final concentration of 5µg/ml overnight at RT.

**Table 2.2**

| **Antibody** | **Source** | **Manufacturer** | **Catalog#** | **Target** |
|---|---|---|---|---|
| IgG1 ctrl | Mouse | BD | 557273 | Background control |
| IgG ctrl | goat | Invitrogen | 026202 | Background control |
| IgG ctrl | rabbit | Invitrogen | 026102 | Background control |
| N-Cadherin | Mouse | BD | 610920 | Proximal tubules |
| E-Cadherin | Mouse | BD | 610182 | Distal tubules |
| Cubilin (A-20) | goat | Santa Cruz | Sc-20609 | Proximal tubules |
| GGT-1 | Rabbit | Santa Cruz | Sc-20638 | Tubular epithelial |
| Megalin | Rabbit | Santa Cruz | Sc-25470 | Proximal tubules |

Labeled NKA constructs were washed twice with 2% goat serum/D-PBS + 0/2% Triton X-100 and incubated with goat or rabbit TRITC conjugated anti-mouse IgG2A (Invitrogen) secondary antibody at 5 µg/ml. For double labeling with DBA (Dolichos biflorus agglutinin), NKA construct candidates were further incubated with FITC conjugated DBA (Vector Labs) diluted to 2 mg/ml in 2% goat serum/D-PBS + 0.2% Triton X-100 for 2hrs at RT. Samples were washed twice with D-PBS and optically sectioned using a Zeiss LSM510 laser scanning confocal system (Cellular Imaging Core, Wake Forest Baptist Medical Center) running LSM Image software (Zeiss) or with a Pathway 855 confocal microscope (BD Biosciences).

**In vivo implantation of acellular biomaterials and NKA Constructs.** Lewis rats (6 to 8 weeks old) were purchased from Charles River (Kalamazoo, MI). All experimental procedures were performed under PHS and IACUC guidelines of the Carolinas Medical Center. Under isoflurane anesthesia, female Lewis rats (approximately 2 to 3 months old) underwent a midline incision, and the left kidney was exposed. 35µl of packed biomaterials (acellular biomaterial or NKA Construct) were introduced by microinjection into the renal parenchyma. Two injection trajectories were used: (i) from each pole toward the cortex (referred to as cortical injection), or (ii) from the renal midline toward the pelvis (referred to as medullary injection). Rats were sacrificed at 1, 4, or 8 weeks post-injection. No early deaths occurred. Study design for the acellular implantation study is presented in Table 33.3 (ND = not done).

**Table 2.3**

| **Study design for evaluating acellular biomaterials in healthy adult Lewis rat kidneys** | | |
|---|---|---|
| **Biomaterial:** | **Time *in vivo*** | |
| | **1 week** | **4 weeks** |
| PCL Beads | *n*=1 | *n*=1 |
| Gelatin Beads | *n*=1 | *ND* |
| Gelatin Particles | *n*=1 | *n*=1 |
| HA/Gelatin Particles | *n*=2 | *ND* |
| HA Particles | *n*=1 | *n*=1 |
| PLGA Particles | *n*=1 | *ND* |
| PLGA Beads | *n*=1 | *ND* |

**Renal Histology.** Representative kidney samples were collected and placed in 10% buffer formalin for 24 hours. Sections were dehydrated in ascending grades of ethanol and embedded in paraffin. Sections (5 µm) were cut, mounted on charged slides, and processed for hematoxylin and eosin (H&E), Masson's trichrome and Periodic Acid Schiff (PAS) staining in accordance with standard staining protocols (Prophet et al., Armed Forces Institute of Pathology: Laboratory methods in histotechnology. Washington, DC: American Registry of Pathology; 1992). Digital microphotographs were captured at total magnification of x40, x100 and x400 using a Nikon Eclipse 50i microscope fitted with a Digital Sight (DS-U1) camera. Renal morphology changes were assessed by commonly used (Shackelford et al. Toxicol Pathol 30(1):93-96; 2002) severity grade schemes (grades 1, 2, 3, 4), to which descriptive terms (minimal, mild, moderate, marked/severe) were applied to describe the degree of glomerulosclerosis, tubular atrophy and dilatation, tubular casts, and interstitial fibrosis, and inflammation observed.

### Results

**Response of mammalian kidney tissue to injection of biomaterials into the renal parenchyma.** Biomaterials were analyzed for potential use in renal cell/biomaterial composites by direct injection into healthy rat kidneys (Table 2.3). Tissue responses were evaluated by measuring the degree of histopathology parameters (inflammation, fibrosis, necrosis, calcification/mineralization) and biocompatibility parameters (biomaterial degradation, neo-vascularization, and neo-tissue formation) at 1 and 4 weeks post-injection.

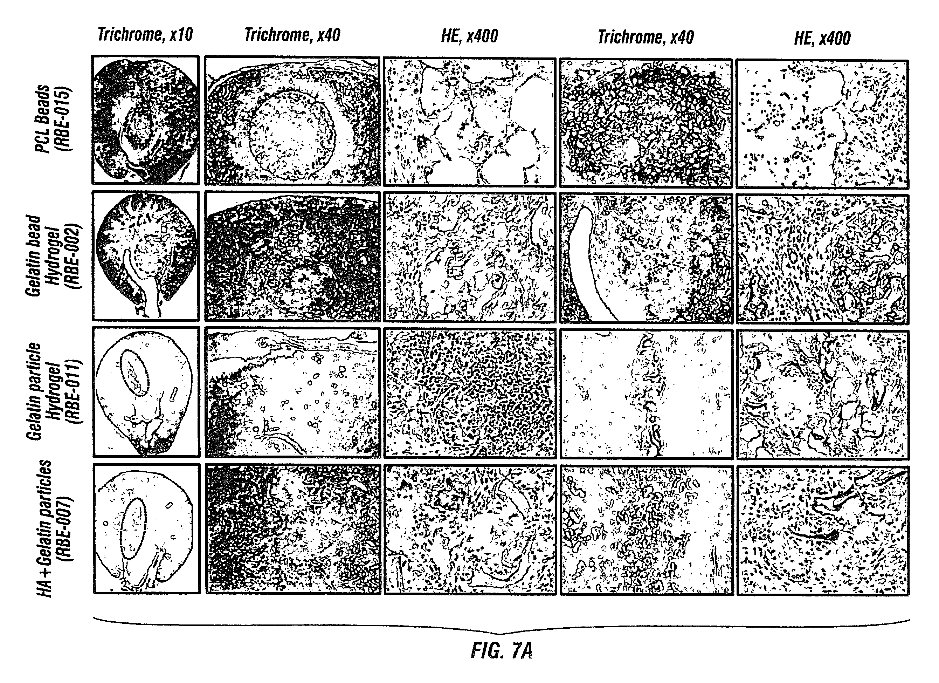

Figure 7A-B shows *in vivo* evaluation of biomaterials at 1 week post-implantation. Trichrome X10 low power image of kidney cross section showing biomaterial aggregate. Trichrome X40: Close-up of biomaterial aggregate. H&E X400: High magnification image of biomaterial aggregate to evaluate extent of cell/tissue infiltration. Each kidney was injected at two locations as described in Materials and Methods. At 1 week post-implantation, the host tissue responses elicited by each biomaterial tested were generally similar; however, gelatin hydrogels appeared to elicit less intense histopathological and more biocompatible responses.

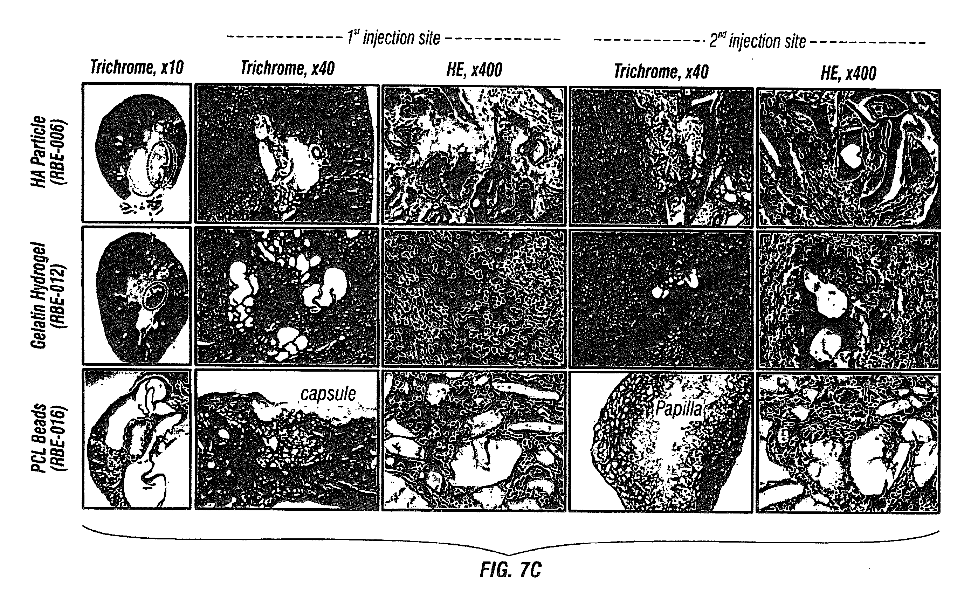

Figure7C shows *in vivo* evaluation of biomaterials at 4 weeks post-implantation. At 4 weeks post-implantation, the severity of histopathology parameters in tissues injected with HA or gelatin particles were qualitatively reduced compared to 1 week post-implantation. Gelatin particles were nearly completely resorbed and less giant cell reaction was observed than in tissues that received HA particles. In most cases where biomaterials were injected via the medullary injection trajectory (e.g., deeper into the medulla/pelvis), undesirable outcomes including obstruction leading to hydronephrosis, inflammatory reactions of greater severity, and renal arteriolar and capillary micro-embolization leading to infarction was observed (data not shown).

**Assessing functional phenotype of therapeutically-relevant renal cell populations with biomaterials.** Therapeutically-relevant renal cell populations (UNFX) that extended survival and increased renal function in a rodent model of chronic kidney disease after direct injection into renal parenchyma have been characterized (Presnell et al. WO/2010/056328; Kelley *et al.* 2010 *supra)* and methods for their isolation, characterization, and expansion have been developed and translated across multiple species (Presnell et al. Tissue Eng Part C Methods. 2010 Oct 27. [Epub ahead of print]). To assess whether UNFX cells adhere to, remain viable, and retain a predominantly tubular, epithelial phenotype when incorporated into NKA Constructs, transcriptomic, secretomic, proteomic, and confocal immunofluorescence microscopy analyses were conducted on NKA Constructs produced from UNFX cells and various biomaterials.

**Adherence and viability.** Canine-derived UNFX cells were seeded with gelatin beads, PCL beads, PLGA beads, HA particles, and HA/gelatin particles as described (3 NKA Constructs per biomaterial). Cell distribution and viability were assessed one day after seeding by live/dead staining.

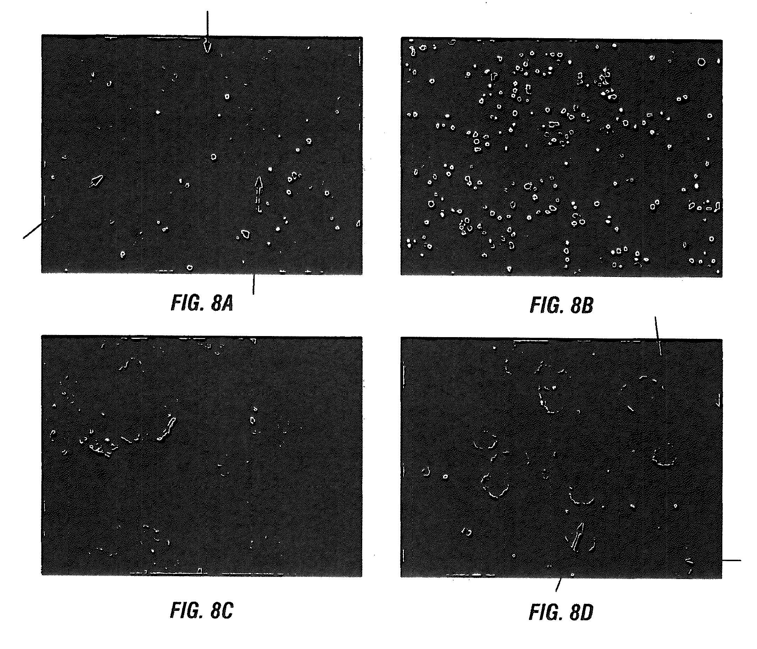

Figure 8A-D shows live/dead staining of NKA constructs seeded with canine UNFX cells (A=gelatin beads; B=PCL beads; C=HA/gelatin particles; D=HA particles). Green indicates live cells; red indicates dead cells. (A) Gelatin beads; (B) PCL beads; (C) HA/gelatin particles; and (D) HA particles. Viable cells may be observed on all hydrogel-based NKA Constructs.

UNFX cells adhered robustly to naturally-derived, hydrogel-based biomaterials such as gelatin beads and HA/gelatin particles (black arrows in A, D), but showed minimal adherence to synthetic PCL (B) or PLGA beads (not shown). Cells did not adhere to HA particles (C) but showed evidence of bioresponse (i.e., spheroid formation). Functional viability of the seeded UNFX cells on hydrogel-based NKA Constructs was confirmed by assaying for leucine aminopeptidase, a proximal tubule-associated hydrolase (data not shown).

**Transcriptomic profiling.** The gene expression profiles of human UNFX cells in hydrogel-based NKA Constructs (3 NKA Constructs per biomaterial) and parallel 2D cultures of UNFX cells were compared by quantitative transcriptomic analysis.

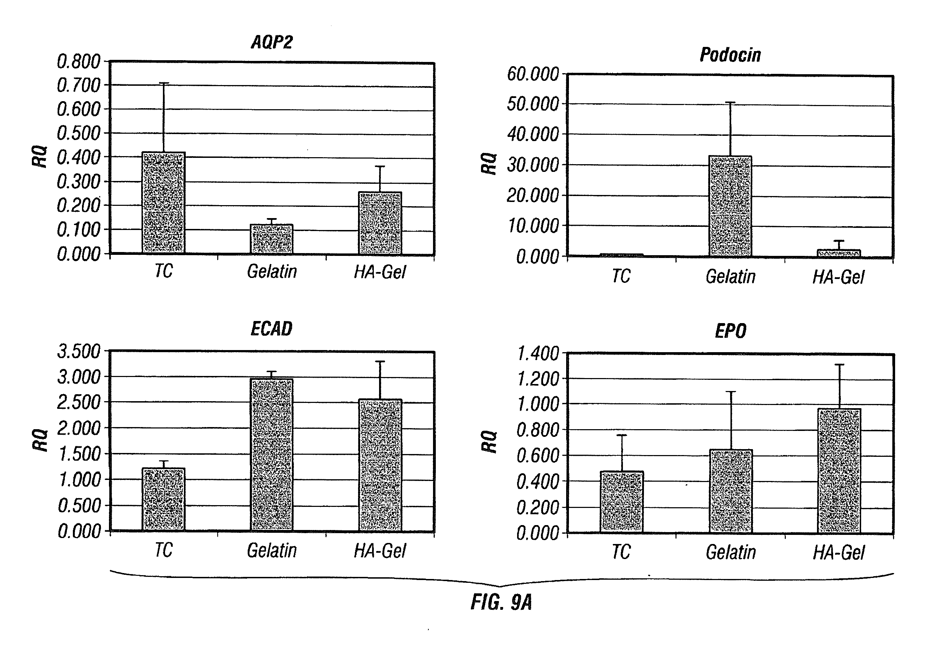

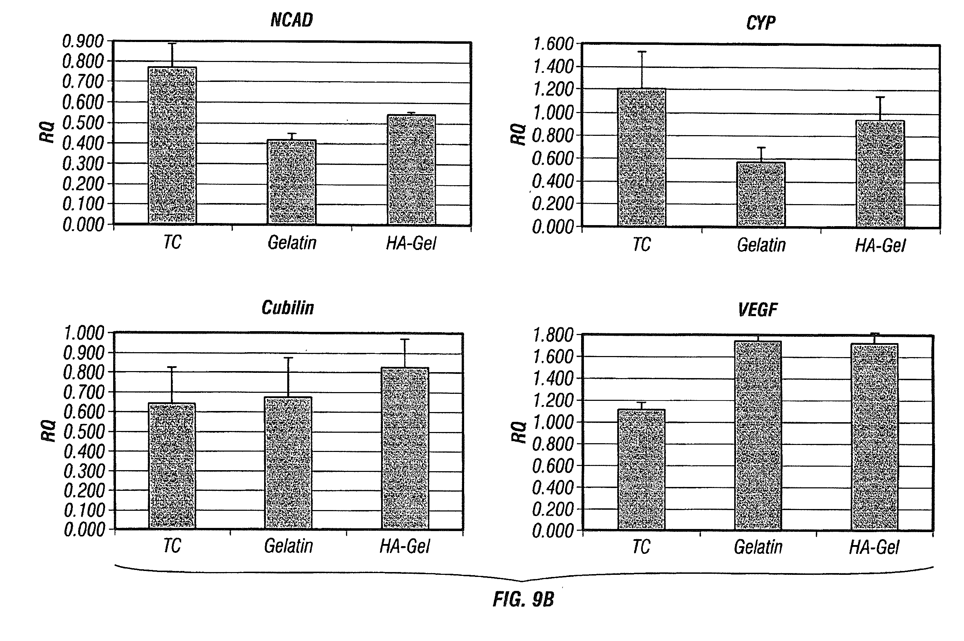

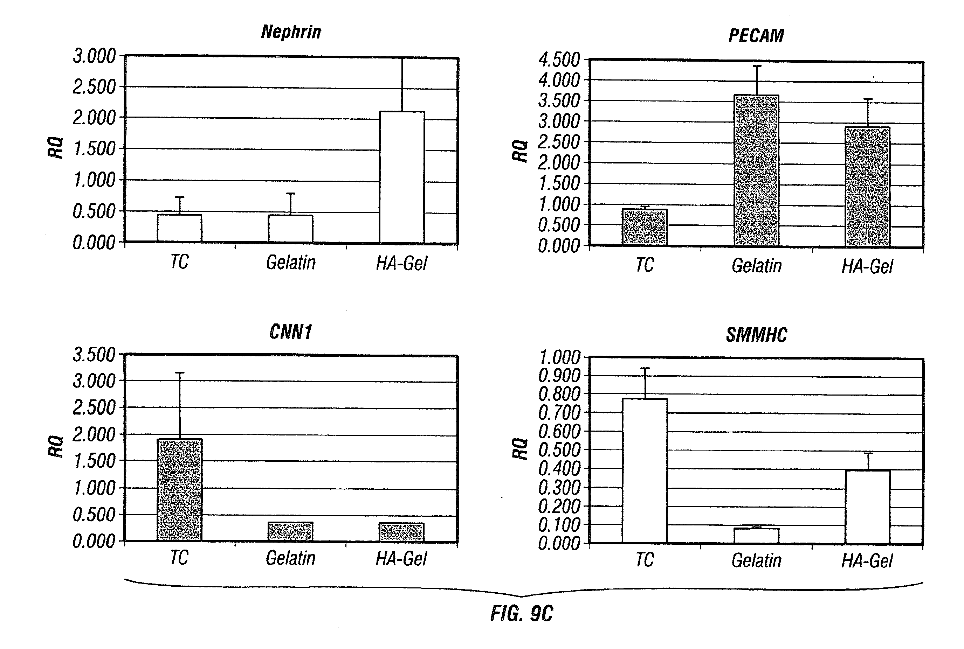

Figure 9A-C shows transcriptomic profiling of NKA constructs. TC: primary human UNFX cells cultured in 2D. Gelatin: NKA Construct composed of human UNFX cells and gelatin hydrogel. HA-Gel: NKA Construct composed of human UNFX cells and HA/gelatin particles. qRT-PCR data presented in graphical and tabular format. Transcripts examined fell into four principal categories: (i) *Tubular*: aquaporin 2(AQ2), E-cadherin (ECAD), erythropoietin (EPO), N-cadherin (NCAD), Cytochrome P450, Family 24, Subfamily A, Polypeptide 1 - aka Vitamin D 24-Hydroxylase (CYP), cubilin, nephrin; (ii) *Mesenchymal:* calponin (CNN1), smooth muscle myosin heavy chain (SMMHC); (iii) *Endothelial*: vascular endothelial growth factor (VEGF), platelet endothelial cell adhesion molecule (PECAM); and (iv) *Glomerular:* podocin. Overall, tubular marker expression was comparable between hydrogel-based NKA Constructs and 2D UNFX cultures. Similarly, endothelial markers (VEGF and PECAM) were comparable. In contrast, the glomerular marker podocin exhibited significant variation among NKA Constructs. Podocin levels in HA/gelatin-based NKA Constructs were most comparable with those observed in 2D UNFX cultures. Interestingly, mesenchymal marker (CNN1 and SMMHC) expression was significantly down-regulated (p < 0.05) in hydrogel-based NKA Constructs relative to 2D UNFX cultures, suggesting that fibroblastic sub-populations of UNFX may not propagate as well in the hydrogel-based NKA Constructs in the renal media formulation. **Secretomic profiling.** NKA Constructs were produced with human UNFX and B2 cells and gelatin or HA/gelatin hydrogel (one NKA Construct per biomaterial per cell type = 4 NKA Constructs total).

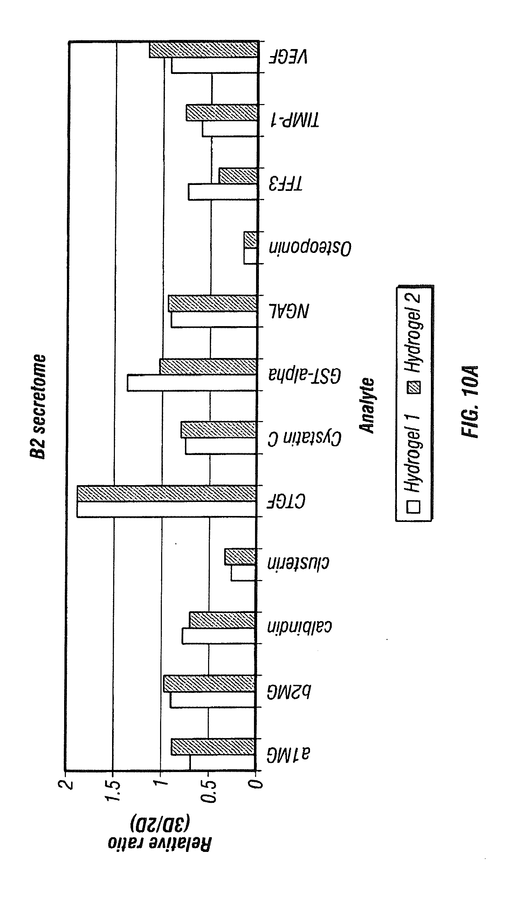

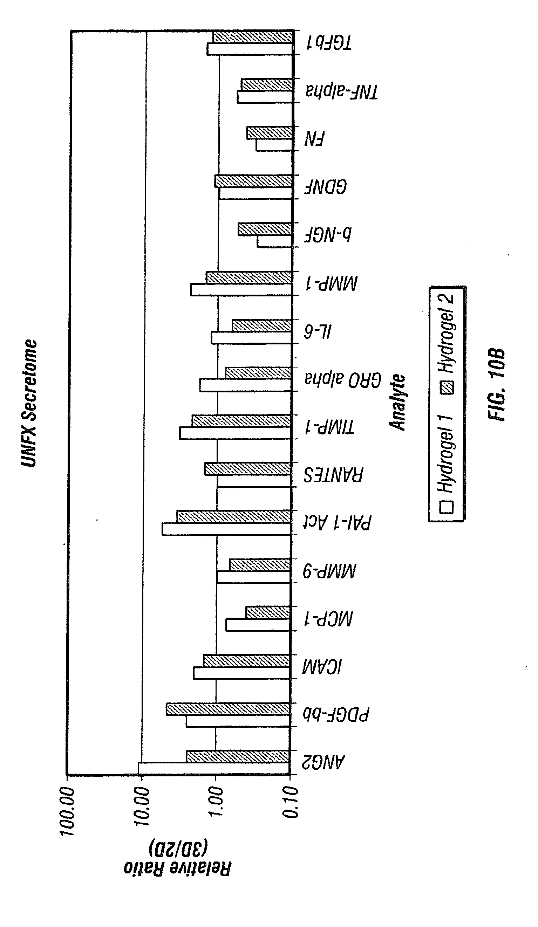

Figure 10A-B shows the secretomic profiling of NKA Constructs. Data is presented as a 3D:2D ratio. NKA Constructs were produced from human UNFX or B2 cells and gelatin (Hydrogel 1) or HA/gelatin (Hydrogel 2) hydrogels as described in Materials and Methods. Secretomic profiling was performed on conditioned media from NKA Constructs matured for 3 days and compared with parallel 2D cultures of human UNFX or B2 cells by calculating the ratio of analyte expression of NKA Constructs (three-dimensional, or 3D, culture) to 2D culture (3D:2D ratio). For each of the three NKA Constructs seeded with UNFX cells, the 3D:2D ratios were at or close to 1, suggesting that the seeding process and 3 days of maturation on these biomaterials had little impact on the secretomic profile of UNFX cells. For NKA Constructs seeded with B2 cells, a similar result of a 3D:2D ratio at or near 1 was observed, providing additional evidence that the seeding process and 3 days of maturation on these biomaterials had little impact on the secretomic profile of therapeutically-relevant renal cells.

**Proteomic profiling.** Proteomic profiles of a given cell or tissue are produced by separating total cellular proteins using 2D gel electrophoresis and have been used to identify specific biomarkers associated with renal disease (Vidal et al. Clin Sci (Lond) 109(5):421-430; 2005).

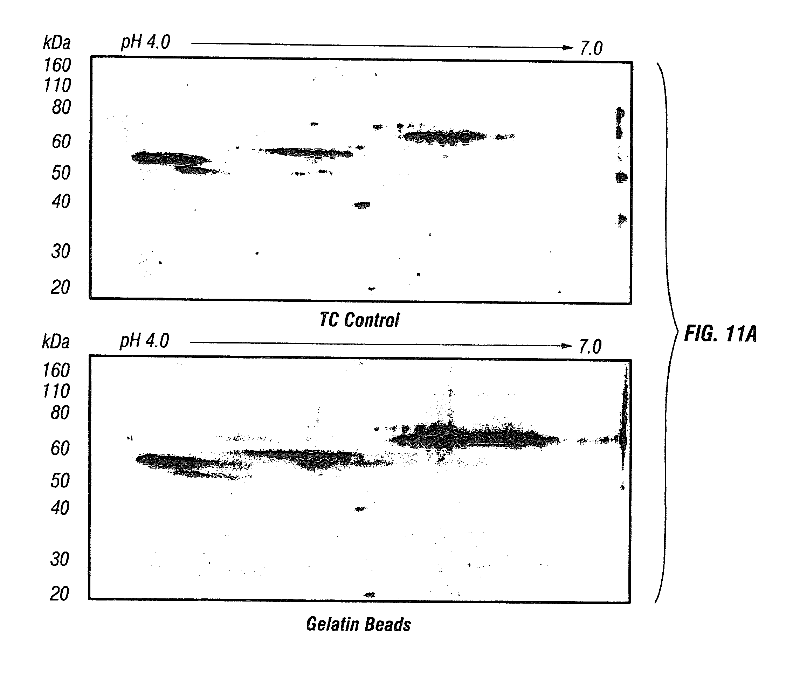

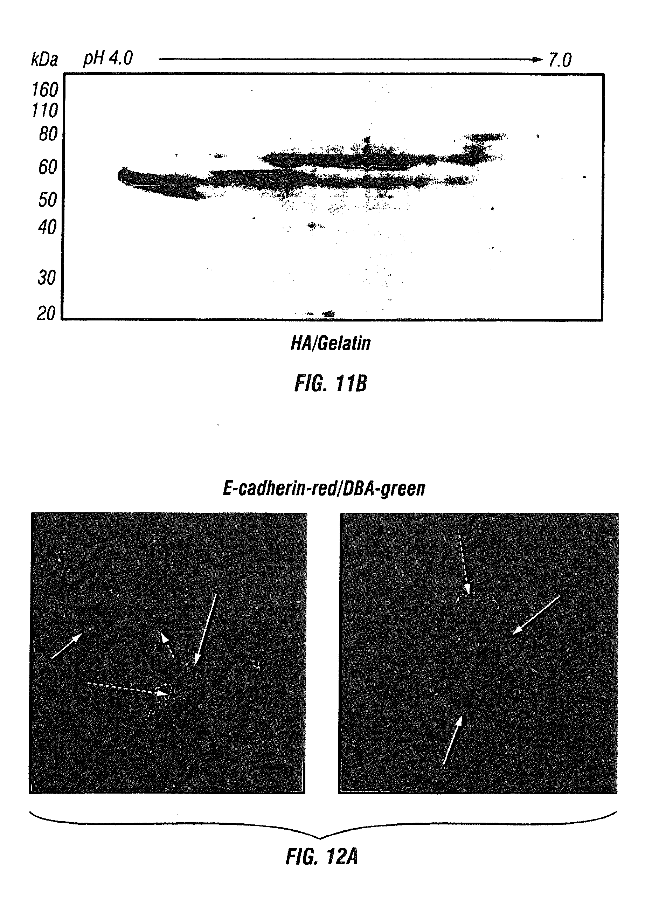

Figure 11A-B shows proteomic profiling of NKA Constructs. NKA Constructs were produced with human UNFX cells and biomaterials as indicated. Proteins in total protein extracts were separated by 2D gel electrophoresis as described in Materials and Methods. In this experiment, proteomic profiling was used to compare protein expression in human UNFX cells in NKA Constructs (gelatin or HA/gelatin hydrogel-based, 3 NKA Constructs per biomaterial) and in 2D tissue culture. The proteome profiles of total protein isolated from NKA Constructs or 2D cultures of UNFX cells were essentially identical, providing additional evidence that the seeding process and 3 days maturation on these biomaterials had little impact on the proteomes expressed by UNFX cells.

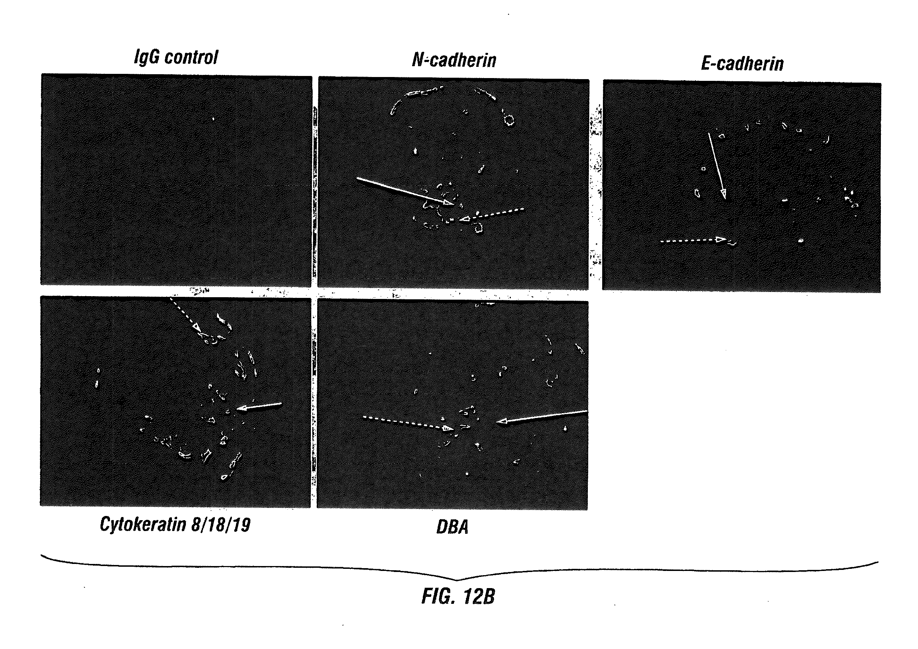

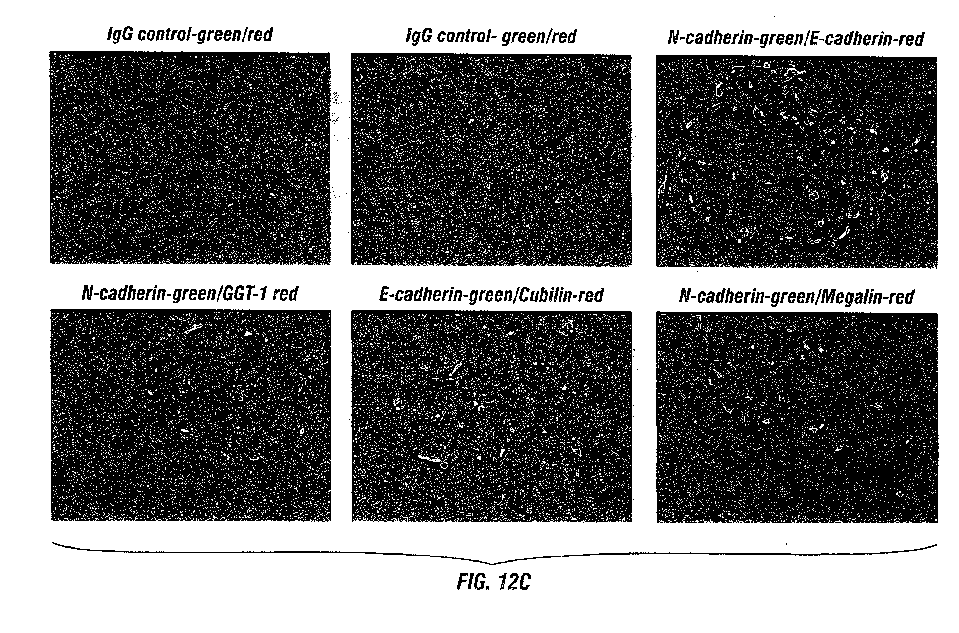

**Confocal microscopy.** Retention of the tubular epithelial phenotype of rat and human B2 cells (Presnell *et al.* 2010 *supra)* in NKA Constructs was evaluated by confocal imaging of established biomarkers: Figure 12A-C shows confocal microscopy of NKA Constructs. Confocal microscopy of NKA Constructs produced with human (A) or rat (B, C) B2 cells and gelatin hydrogel. (A) E-cadherin (red - solid white arrows), DBA (green - dashed white arrows) and gelatin hydrogel bead is visible with DIC optics. (B) DNA visualized with DAPI staining (blue - solid white arrows) and each of the following markers in green (dashed white arrows): IgG control, N-cadherin, E-cadherin, cytokeratin 8/18/18, DBA. (C) double-labeling images of markers and colors as indicated. E-cadherin and DBA in human NKA Constructs and E-cadherin, DBA, N-cadherin, cytokeratin 8/18/19, gamma glutamyl transpeptidase (GGT-1), and megalin in rat NKA Constructs. Optical sectioning of confocal images also allowed evaluation of the extent of cell infiltration into the biomaterial after seeding and 3 days of maturation. B2 cells in human and rat NKA Constructs exhibited expression of multiple tubular epithelial markers. Optical sectioning revealed minimal cell infiltration of the hydrogel construct, with cells generally confined to the surface of the biomaterial.

**In vivo responses to implantation of NKA construct prototypes.** Based on *the in vivo* responses to biomaterial injection into renal parenchyma and the *in vitro* phenotype and functional characterization of UNFX and B2 cells in NKA Constructs described above, gelatin hydrogel was selected to evaluate the *in vivo* response to NKA Construct injection into renal parenchyma in healthy Lewis rats. NKA Constructs were produced from syngeneic B2 cells and implanted into two animals, which were sacrificed at 1, 4, and 8 weeks post-implantation. All animals survived to scheduled necropsy when sections of renal tissues were harvested, sectioned, and stained with Trichrome, hematoxylin and eosin (H&E), and Periodic Acid Schiff (PAS).

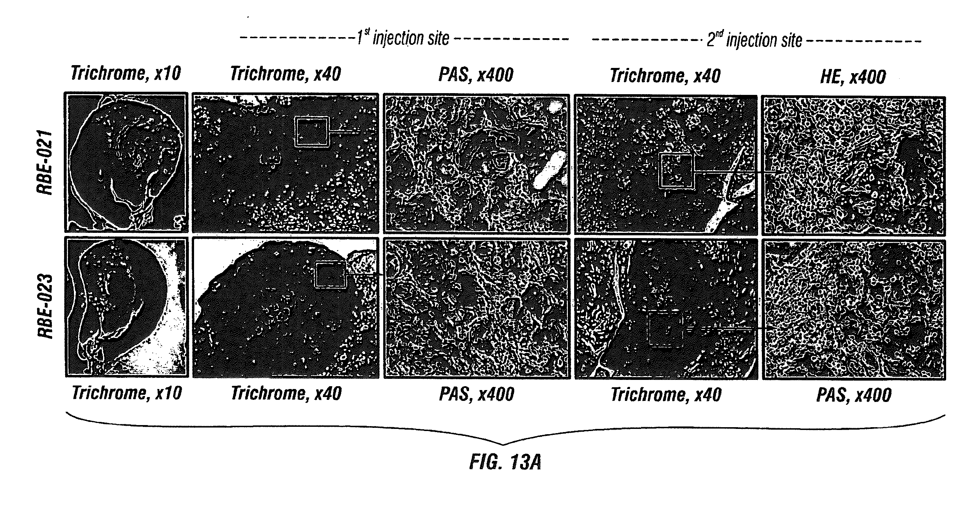

Figure 13A-B shows *in vivo* evaluation of NKA Constructs at 1 and 4 weeks post-implantation. Trichrome X10 low power image of kidney cross section showing biomaterial aggregate. Trichrome X40: Close-up of biomaterial aggregate. H&E/PAS X400: High magnification image of biomaterial aggregate to evaluate extent of cell/tissue infiltration. Each kidney was injected at two locations as described in Materials and Methods.

Figure 13A shows *in vivo* evaluation of NKA Constructs at 1 week post-implantation. At 1 week post injection, gelatin beads were present as focal aggregates (left panel, circled area) of spherical and porous material staining basophilic and surrounded by marked fibro-vascular tissue and phagocytic multi-nucleated macrophages and giant cells. Fibrovascular tissue was integrated within the beads and displayed tubular epithelial components indicative of neo-kidney tissue formation. Additionally, tubular and vasculoglomerular structures were identified by morphology (PAS panels).

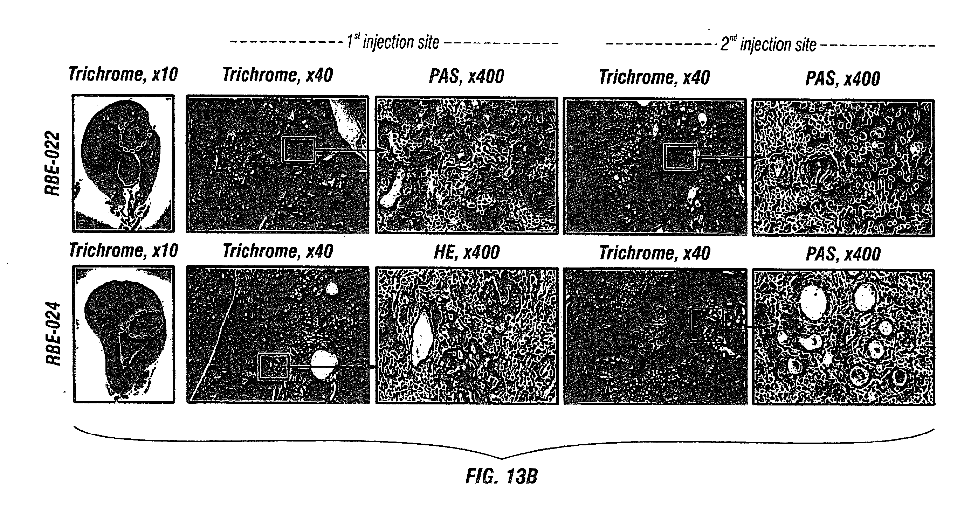

Figure 13B shows *in vivo* evaluation of NKA Constructs at 4 weeks post-implantation. By 4 weeks post-injection, the hydrogel was completely resorbed and the space replaced by progressive renal regeneration and repair with minimal fibrosis (note the numerous functional tubules within circled area of 4-week Trichrome panel).

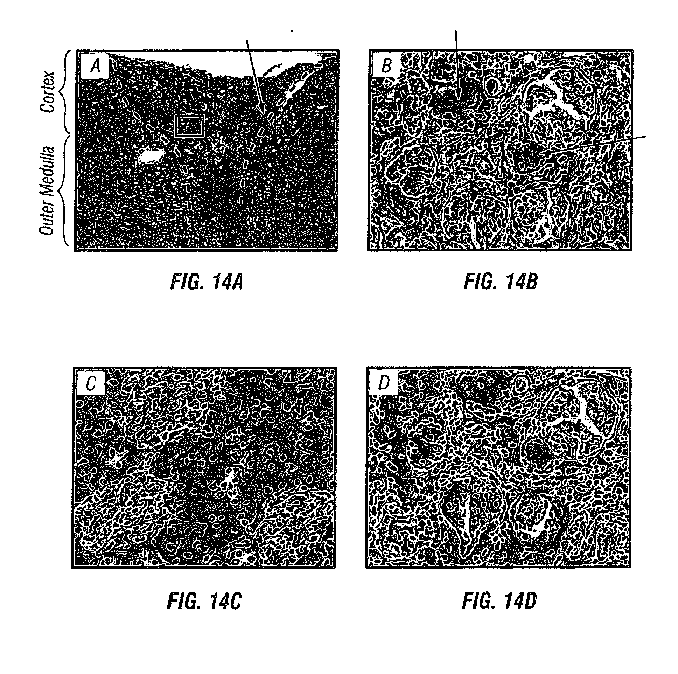

Figure 14A-D shows *in vivo* evaluation of NKA Construct at 8 weeks post-implantation. Trichrome X10 low power image of kidney cross section showing biomaterial aggregate. Trichrome X40: Close-up of biomaterial aggregate. H&E/PAS X400: High magnification image of biomaterial aggregate to evaluate extent of cell/tissue infiltration. (A) Moderate chronic inflammation (macrophages, plasma cells and lymphocytes), moderate numbers of hemosiderin-laden macrophages (chronic hemorrhage due to injection) with marked fibrovascular response (blue stained by Masson's trichrome - black arrows).; (B) Higher magnification (trichrome stained, x400) of boxed area of (A) showing regenerative response induction consistent with neo-kidney tissue formation (C) Representative of adjacent (normal) kidney parenchyma showing typical cortical glomeruli morphology HE, x400); (D) HE stained section, x400 comparing new glomeruli morphology observed in treatment area vs. Figure 14C.

Figure 14A-D shows *in vivo* evaluation of NKA Construct at 8 weeks post-implantation. At 8 weeks post-implantation, evidence of neo-kidney like tissue formation was observed, consistent with induction of early events in nephrogenesis. Comparison of the area of regenerative induction (B, D) with adjacent cortical parenchyma (C) showed presence of multiple S-shaped bodies and newly formed glomeruli.

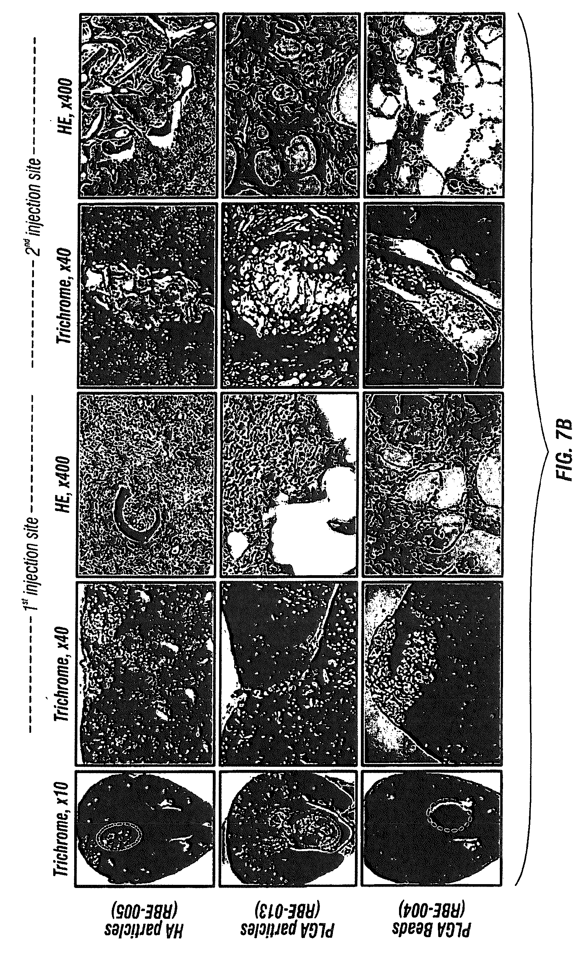

This study investigated the responses of mammalian renal parenchyma to implantation of synthetic and natural biomaterials, both acellular and as bioactive renal cell/biomaterial composites (i.e., NKA Constructs). A combination of *in vitro* functional assays and *in vivo* regenerative outcomes were analyzed to functionally screen candidate biomaterials for potential incorporation into a NKA construct prototype. Implantation of acellular hydrogel-based biomaterials into renal parenchyma (Figure 7) was typically associated with minimal fibrosis or chronic inflammation and no evidence of necrosis by 4 weeks post-implantation. Moderate cellular/ tissue in-growth and neo-vascularization was observed, with minimal remnant biomaterial. Based on these *in vivo* data, hydrogel-based biomaterials were selected to produce NKA Constructs with which to evaluate *in vitro* biofunctionality and *in vivo* regenerative potential. *In vitro* confirmation of material biocompatibility was provided through live/dead analysis of NKA Constructs (Figure 8). Gelatin-containing hydrogels were associated with robust adherence of primary renal cell populations. Phenotypic and functional analysis of NKA Constructs produced from bioactive primary renal cell populations (UNFX or B2) and hydrogel biomaterials was consistent with continued maintenance of a tubular epithelial cell phenotype. Transcriptomic, secretomic, proteomic, and confocal microscopy analyses of NKA Construct confirmed no significant differences relative to primary renal cells seeded in 2D culture. Finally, implantation of hydrogel-based NKA construct into the renal parenchyma of healthy adult rodents was associated with minimal inflammatory and fibrotic response and regeneration of neo-kidney like tissue by 8 weeks post-implantation.

Taken together, these data provide evidence suggesting that a regenerative response was induced *in vivo* by NKA Constructs. These studies represent the first *in vivo,* intra-renal investigations of the biological response of mammalian kidney to implantation of a therapeutically-relevant primary renal cell/biomaterial composite. Observed results are suggestive that NKA Constructs have the potential to both facilitate regeneration of neo-kidney tissue and attenuate non-regenerative (e.g., reparative healing) responses.

Throughout the foregoing description the invention has been discussed with reference to certain embodiments, but it is not so limited. Indeed, various modifications of the invention in addition to those shown and described herein will become apparent to those skilled in the art from the foregoing description and fall within the scope of the appended claims

Furthermore, the present invention relates to the following items:
1. A method for identifying inputs necessary to elicit a regenerative response in a target tissue in need of regeneration, comprising
   (a) identifying functional elements of a reference tissue of the same type;
   (b) identifying functional deficits or abnormalities associated with the target tissue;
   (c) isolating the functional elements of the reference tissue, individually or as controlled admixtures;
   (d) creating a core list of putative inputs wherein a plurality of functional elements is identified in step (a);
   (e) creating a test grid comprising all combinations of putative inputs from the core list; and
   (f) conducting *in vivo* experiments based on the test grid to ascertain the target tissue functional response to the combinations tested; and
   (g) based on the results of the *in vivo* experiments, identifying the inputs capable of modulating a regenerative response in the target tissue.
2. The method of item 1 wherein the core list of putative inputs created in step (d) is supplemented by additional inputs.
3. The method of item 1 wherein prior to inclusion in *the in vivo* experiments in step (f), one or more putative inputs are tested individually to determine whether said input has a negative effect on said target tissue or components thereof.
4. The method of item 3 wherein said testing is performed *in vivo.*
5. The method of item 3 wherein said testing is performed *in vitro.*
6. The method of item 3 wherein if a negative effect is determined, said input excluded from the *in vivo* experiments in step (f).
7. The method of item 3 wherein if a neutral or positive effect is determined, said input is included in *the in vivo* experiments in step (f).
8. The method of item 1 wherein the target tissue comprises multiple cellular compartments.
9. The method of item 8 wherein in step (f) function of each cellular compartment is tested.
10. The method of item 1 further comprising the step of one or more multivariate analysis of inputs identified.
11. The method of item 1 wherein one or more inputs identified are cellular components of a regenerative stimulus.
12. The method of item 11 wherein said cellular components provide direct function *in vivo.*
13. The method of item 11 wherein said cellular components provide indirect stimulation of endogenous elements of the target tissue upon *in vivo* delivery.
14. The method of item 11 wherein said cellular components are derived from tissue resident cells.
15. The method of item 11 wherein said cellular components are not derived from tissue resident cells.
16. The method of item 15 wherein the non-tissue resident cells are obtained from a source that differs from the source of the target tissue.
17. The method of item 14 wherein said tissue resident cells are autologous.
18. The method of item 14 wherein said tissue resident cells are allogeneic.
19. The method of item 14 wherein said tissue resident cells are fully differentiated, partially differentiated, or undifferentiated.
20. The method of item 1 wherein at least some of the inputs identified are biomaterials.
21. The method of item 20 wherein at least some of the biomaterials facilitate a regenerative response.
22. The method of item 20 wherein at least some of the biomaterials direct the form or function of cells through national or engineered biological or biophysical properties.
23. The method of item 1 wherein at least some of the inputs identified are bioactive molecules.
24. The method of item 23 wherein said bioactive molecules comprise cytokines and growth factors.

## Claims

1. A method for identifying inputs necessary to elicit a regenerative response in a kidney or liver in need of regeneration, comprising
(a) identifying cellular components of a reference kidney or liver;
(b) identifying functional deficits or abnormalities associated with the degeneration of the kidney or liver in need of regeneration;
(c) isolating the cellular components of the reference kidney or liver, individually or as a combination of two or more isolated, enriched cell populations derived from an unfractionated, heterogenous cell population;
(d) creating a core list of putative inputs selected from the cellular components identified in step (a), wherein said core list is optionally supplemented with additional inputs selected from the group of other cellular components, biomaterial and bioactive molecule elements;
(e) creating a test grid comprising all combinations of putative inputs from the core list;
(f) conducting *in vivo* experiments with at least a subset of combinations of the test grid in step (e) in non-human animals to ascertain the functional response of the kidney or liver to the combinations tested;
(g) based on the results of *the in vivo* experiments, identifying the inputs capable of modulating a regenerative response in the kidney or liver in need of regeneration; and
(h) performing one or more multivariate analysis of inputs identified in step (g).

2. The method of claim 1, wherein the reference kidney or liver is a healthy reference kidney or liver, or wherein the reference kidney or liver is a non-healthy reference kidney or liver.

3. The method of any one of claims 1 or 2, wherein at least some of the biomaterials facilitate a regenerative response or direct the form or function of cells through natural or engineered biological or biophysical properties.

4. The method of any one of claims 1 to 3, wherein the biomaterial is a biocompatible polymeric matrix or scaffold.

5. The method of any one of claims 1 to 4, wherein the bioactive molecule elements are selected from the group consisting of cytokines, growth factors, pharmacological agents, peptides, proteins and chemical moieties.

6. The method of any one of claims 1 to 5, wherein the deficits or abnormalities associated with degeneration of the liver or kidney in need of regeneration are known cellular, compositional, and/or structural deficits or abnormalities associated with degeneration of the liver or kidney in need of regeneration.

7. The method of any one of claims 1 to 6, wherein prior to inclusion in the experiments in step (f), one or more putative inputs are tested individually to determine whether said input has a negative effect on said target tissue, organ or components thereof.

8. The method of claim 7, wherein if a negative effect is determined, said input is excluded from the experiments in step (f), or if a neutral or positive effect is determined, said input is included in the experiments in step (f).

9. The method of any one of claims 1 to 8, wherein in step (f) function of each cellular compartment is tested.

10. The method of claim 1, wherein the cellular components are selected from tubular cell compartment, glomerular compartment, vascular compartment, endocrine compartment and collecting duct system.

11. The method of claim 10, wherein the cellular components comprise tubular cells (proximal); tubular cells (distal); collecting duct cells; vascular cells (afferent / efferent arterioles, endothelial cells, vascular smooth muscle cells); erythropoietin-producing interstitial fibroblasts; other interstitial cells; resident progenitors (various); glomerular cells (podocytes, mesangial, endothelial); specific regional architecture (cortical, cortico-medullary, medullary, calyx); ionic and oxygen gradients (spatial); tubular basement membrane (laminin, collagen IV, perlecan); glomerular basement membrane (collagen IV, laminin, nidogen, and heparin sulfate proteoglycans); and renal pelvis.

12. The method of claim 11, wherein the functional deficits or abnormalities are selected from proteinuria, albuminuria, hypercholesterolemia, increased blood urea nitrogen, increased blood creatinine, tubular degeneration, tubular atrophy, tubular luminal dilatation with cellular debris and proteinaceous casts, tubulo-interstitial fibrosis, glomerular hypertrophy, glomerular atrophy, glomerular sclerosis, epo-deficiency, anemia, vitamin D deficiencies, disturbances in the renin-angiotensin system, hypertension, altered tubular-glomerular feedback mechanisms, inflammatory aggregates with interstitial fibrosis, interstitial fibrosis, and the epithelial-mesenchymal-transformation of collecting duct epithelium.

13. The method of claim 1, wherein said cellular components are derived from tissue resident cells or are not derived from tissue resident cells.

14. The method of claim 13, wherein said tissue resident cells are autologous or allogeneic.

15. The method of claim 13, wherein said tissue resident cells are fully differentiated, partially differentiated, or undifferentiated.
